# EUROPÄISCHE PATENTSCHRIFT

(11) **EP 1 294 837 B1**
(45) Veröffentlichungstag und Bekanntmachung des Hinweises auf die Patenterteilung: **24.08.2005**
(21) Anmeldenummer: 01960307.5
(22) Anmeldetag: 13.06.2001
(51) Int. Cl.: C11D 1/72, C11D 1/14, C11D 1/29, C11D 1/34, C07C 29/16, C07C 303/24, C07F 9/09

(54) **TENSIDE AUF DER BASIS VON OXOALKOHOLEN**
OXOALCOHOL-BASED DETERGENT
TENSIOACTIFS A BASE D'ALCOOLS OXO

(30) Priorität: 16.06.2000 DE 10029692; 16.06.2000 DE 10029693
(43) Veröffentlichungstag der Anmeldung: 26.03.2003
(73) Patentinhaber: BASF Aktiengesellschaft, 67056 Ludwigshafen (DE)
(72) Erfinder: TROPSCH, Jürgen, 67354 Römerberg (DE); MAAS, Heiko, 68165 Mannheim (DE)
(74) Vertreter: Isenbruck, Günter
(86) Internationale Anmeldenummer: PCT/EP2001/006709
(87) Internationale Veröffentlichungsnummer: WO 2001/096508

(56) Entgegenhaltungen:
- WO-A-00/39058
- WO-A-97/38957
- WO-A-97/46311
- WO-A-99/19440

## Beschreibung

Die vorliegende Erfindung betrifft Wasch- und Reinigungsmittel, die einen wirksamen Anteil von Niotensiden oder anionischen Tensiden auf Basis von Oxoalkoholen mit 11 bis 13 C-Atomen enthalten, welche aufgrund ihrer Struktur nächstvergleichbaren bekannten Tensiden deutlich überlegen sind. Die Erfindung betrifft ferner Verfahren zur Herstellung von Wasch- und Reinigungsmitteln unter Einsatz besagter Tenside sowie die Verwendung der erfindungsgemäßen Wasch- und Reinigungsmittel.

Waschmittel im Sinne dieser Erfindung dienen in der Regel zum Waschen von mehr oder weniger flexiblen Materialien, vorzugsweise solchen, die natürliche, synthetische oder halbsynthetische Fasermaterialien enthalten oder daraus bestehen und die demzufolge zumindest teilweise einen textilen Charakter aufweisen.

Waschmittel dieser Art sind im Stand der Technik vielfach beschrieben worden. Einen sehr guten Überblick über die Wirkungsweise und die Zusammensetzung von Waschmitteln findet man beispielsweise in Ullmann's Encyclopedia of Industrial Chemistry, 5. Auflage, Bd. A8, (1986), Seiten 315 ff, Stichwort "Detergents".

Reinigungsmittel im Sinne dieser Erfindung dienen in der Regel zur Reinigung von Oberflächen, die keine oder nur wenige und kleine Poren haben und infolgedessen keine oder nur eine geringe Saugfähigkeit aufweisen.

Reinigungsmittel dieser Art sind im Stand der Technik vielfach beschrieben worden. Einen sehr guten Überblick über die Wirkungsweise und die Zusammensetzung von Reinigungsmitteln findet man beispielsweise in Ullmann's Encyclopedia of Industrial Chemistry, 5. Auflage, Bd. A7, (1986), Seiten 137 ff.

Die Wasch- und Reinigungsmittel enthalten ein Tensid oder mehrere Tenside aus gleichen oder unterschiedlichen Tensidgruppen und in der Regel weitere Hilfs- und Zusatzstoffe, die entweder zur Konfektionierung erforderlich sind und/oder die einer Anpassung der Wasch- und Reinigungsmittel an den geplanten speziellen Verwendungszweck oder die Art der Anwendung (Wäsche bzw. Reinigung von Harid oder in Maschinen) dienen. Bestandteile, die neben den verschiedenen Tensiden in wechselnden Kombinationen und Anteilen in vielen Wasch- und Reinigungsmitteln eingesetzt werden können, sind zum Beispiel Builder (Sequestrierungsmittel) und Co-Builder, pH-Regulatoren, wie anorganische oder organische Säuren, anorganische oder organische Basen und Puffersysteme, Dispergiermittel, Schmutztragemittel, Verdickungsmittel, Enzyme, Bleichsysteme, hydrotrope Verbindungen als Lösungsvermittler bzw. Solubilisatoren, wie z.B. Harnstoff oder Alkohole, Schaumregulatoren zur Stabilisierung oder Dämpfung des Schaums, Haut- und Korrosionsschutzmittel, desinfizierende Verbindungen oder Systeme, beispielsweise solche, die Jod enthalten oder die Chlor oder unterchlorige Säure freisetzen, wie z.B. Dichlorisocyanurat, Parfüm, Farbstoffe, und Biozide.

Waschmittel enthalten darüber hinaus gegebenenfalls noch optische (fluoreszierende) Aufheller, Vergrauungsinhibitoren, sowie Stell- und Konfektionierungsmittel, in Reinigungsmitteln werden gegebenenfalls zusätzlich organische Lösemittel und feinteilige Abrasivkomponenten, wie z.B. Quarz- oder Mairmormehl, Kreide, Diatomeenerde, Bimsstein, Polierrot oder Schmirgel, eingearbeitet.

Wesentlichen Anteil an der Reinigungswirkung der im Stand der Technik beschriebenen Wasch- und Reinigungsmittel kommt den darin enthaltenen Tensiden zu. Verwendung finden ionische Tenside und zwar sowohl anionische wie beispielsweise Alkoholsulfate, Alkoholethersulfate, Alkylbenzolsulfonate, α-Olefinsulfonate, Sulfosuccinate als auch kationische Tenside, wie beispielsweise C₈ bis C₁₆-Dialkyldimethylammoniumhalogenide, Dialkoxydimethylammoniumhalogenide oder Imidazoliniumsalze mit langkettigem Alkylrest.

Auch der Einsatz von amphoteren Tensiden, beispielsweise von Derivaten von sekundären oder tertiären Aminen wie z.B. C₆-C₁₈-Alkylbetainen oder C₆-C₁₅-Alkylsulfobetainen oder Aminoxiden wie Alkyldimethylaminoxiden ist bereits beschrieben worden.

Auch nichtionische Tenside, insbesondere auch Alkoxylate und Polyglycoside von längerkettigen und langkettigen Alkanolen insbesondere mit 8 bis 20 C-Atomen sowie Alkoxylaten von Alkylaminen und Alkylamiden werden in Wasch- und Reinigungsmitteln eingesetzt. Es ist insbesondere auch bekannt, Oxoalkohole mit 10 bis 13 C-Atomen in Form ihrer Phosporsäure- oder Schwefelsäureester sowie Alkoxylate dieser Oxoalkohole direkt oder in Form ihrer Phosporsäure- oder Schwefelsäureester als Tenside in Wasch- und Reinigungsmitteln einzusetzen.
So ist es aus der WO 97/38957 bekannt, verzweigte, gegebenenfalls oxalkylierte Alkohole und deren Sulfate herzustellen durch Dimerisierung von α-Olefinen mit 5 bis 9 C-Atomen, Isomerisierung der erhaltenen Vinyliden-Dimergemische zu Gemischen interner Olefine, und Hydroformylierung zu den gewünschten Alkoholen, die, gegebenenfalls nach Oxethylierung, in die Sulfate überführt werden. Die so erhaltenen Produkte können, gegebenenfalls in Kombination mit anderen Tensiden, in Wasch- und Reinigungsmitteln eingesetzt werden.
Die WO 97/46311 beschreibt die Verwendung von blockförmigen iso-Tridecanolalkoxylaten als schaumarme oder schaumdämpfende Tenside in Wasch- und Reinigungsformulierungen. Das iso-Tridecanol wird durch katalytische Oligomerisierung niederer Olefine, beispielsweise durch Tetramerisierung von Propylen, Trimerisierung von C₄-Olefinen oder durch Dimerisierung von 2-Methyl-1-penten und anschließende Hydroformylierung an einem Oxo-Katalysator hergestellt.

Aus der WO 00/39058 ist ein Verfahren bekannt zur Herstellung von neuen Tensidalkoholen und Tensidalkoholethern durch Derivatisierung von Olefinen mit etwa 10 bis 20 C-Atomen oder von Gemischen solcher Olefine zu Alkanolen und gegebenenfalls anschließende Alkoxylierung. Bei diesem Verfahren wird ein C₄-Olefin-Gemisch der Metathese unterworfen, die erhaltenen Olefine werden dimerisiert, und anschließend zu Tensidalkoholen derivatisiert und diese gegebenenfalls alkoxyliert. Das bei der Dimerisierung erhaltene Olefingemisch enthält einen hohen Anteil verzweigter Komponenten und weniger als 10 Gew.-% von Verbindungen, die eine Vinylidengruppe enthalten. Beschrieben wird ferner die Verwendung der Tensidalkohole und Tensidalkoholether zu Tensiden durch Glycosidierung oder Polyglycosidierung, Sulfatierung oder Phosphatierung.

Im Interesse eines möglichst sparsamen Stoffeinsatzes, hoher Wirtschaftlichkeit und last not least geringer Umweltbelastung streben die Hersteller von Wasch- und Reinigungsmitteln nach einer stetigen Verbesserung der Wirksamkeit ihrer Produkte und insbesondere der darin enthaltenen Tenside.

Es wurde nun überraschend gefunden, daß die unten beschriebenen erfindungsgemäßen Wasch- und Reinigungsmittel gegenüber nächst vergleichbaren bekannten Mitteln eine erheblich überlegene Wirksamkeit entfalten.

Ein Gegenstand der vorliegenden Erfindung sind somit Wasch- und Reinigungsmittel, umfassend einen wirksamen Anteil eines oder mehrerer Tenside der Formel I 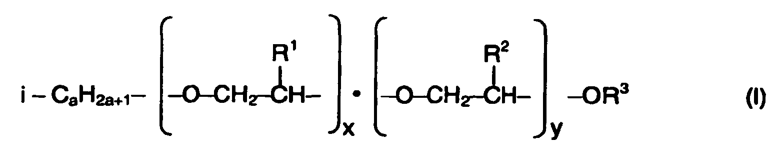 worin
a für eine der Zahlen 11, 12 und 13 steht, wobei der i - Cₐ H₂ₐ₊₁- Rest von einem Oxoalkohol abgeleitet ist, der durch Hydroformylierung eines Decens, Undecens, Dodecens oder eines Gemisches dieser Olefine erhalten wird, das seinerseits durch Dimerisierung von n-Penten-2, Hexen-3 oder von Gemischen dieser Verbindungen hergestellt worden ist,
R¹ und R² verschieden sind und jeweils Wasserstoff oder Alkylreste der Formel CₙH₂ₙ₊₁-bedeuten,
R³ Wasserstoff, oder einen Sulfato- oder Phosphatorest bedeutet,
n für eine Zahl von 1 bis 16, x für eine Zahl von 0 bis 200 und y für eine Zahl von 0 bis 200 steht,
sowie weitere in Wasch- und Reinigungsmitteln übliche bekannte Hilfs- und Zusatzstoffe und
gegebenenfalls zusätzlich weitere Tenside.

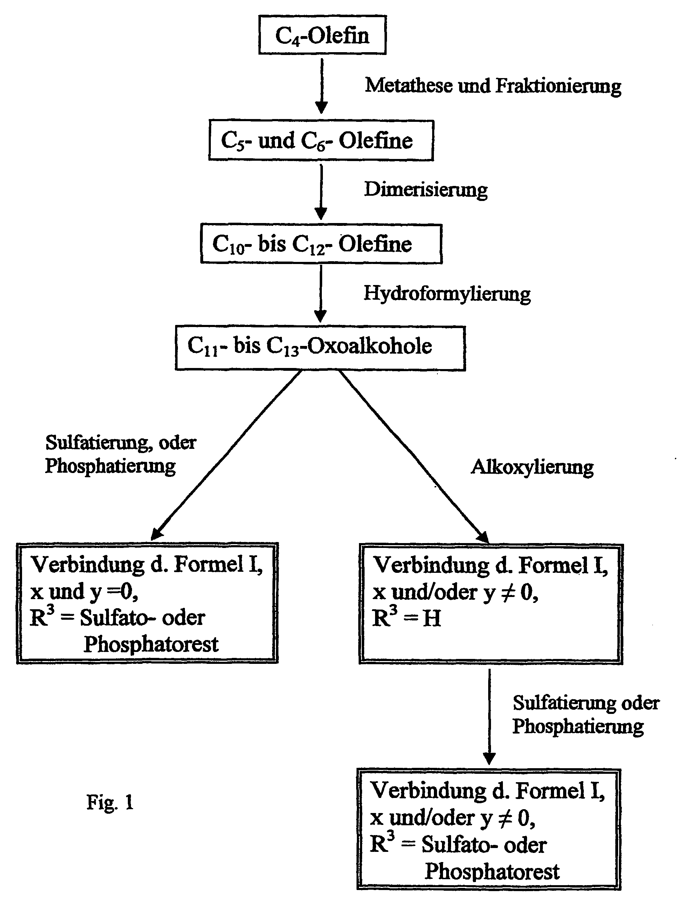

In der folgenden Erfindungsbeschreibung und den Ausführungsbeispielen wird auf Figuren 1 bis 3 Bezug genommen.

Die Figur 1 veranschaulicht den zu den erfindungsgemäßen Wasch- und Reinigungsmittel führenden Syntheseweg,

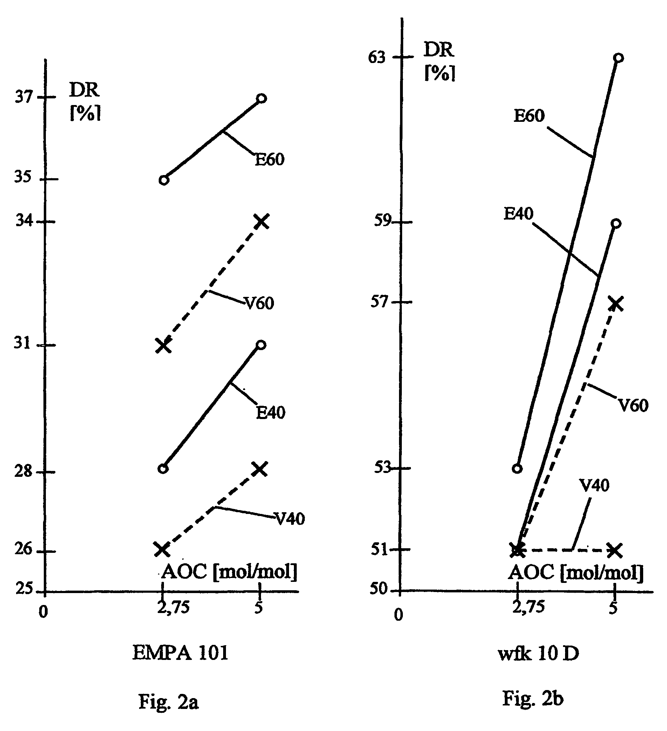

die Figuren 2a und 2b veranschaulichen die Waschwirkung von erfindungsgemäßen und strukturell vergleichbaren, nicht erfindungsgemäßen Waschmitteln, jeweils mit variierten Alkylenoxid-Gehalten am Beispiel der Wäsche von zwei Geweben mit unterschiedlicher Standard-Anschmutzung, wobei die Steigerung des Remissionsgrades als Maß für die Waschwirkung dient,

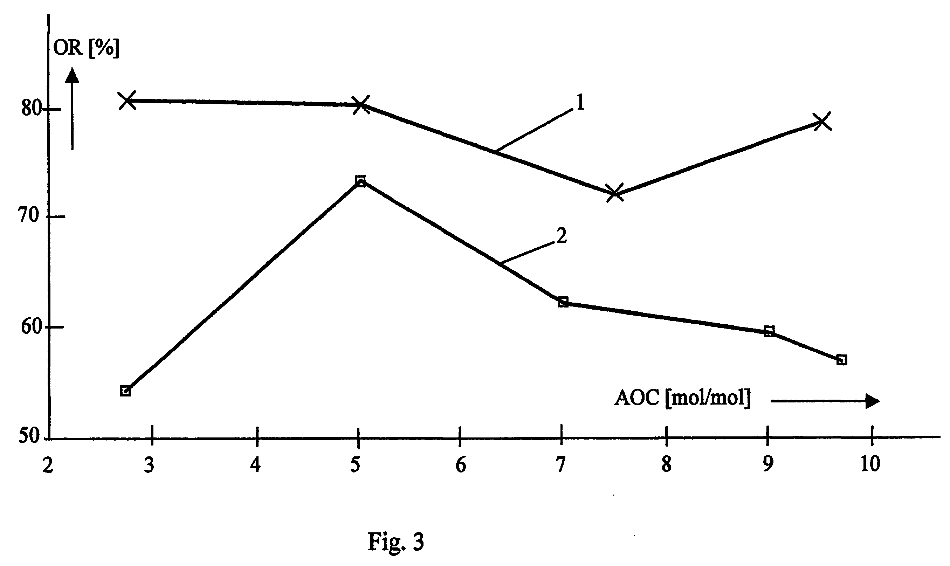

die Figur 3 veranschaulicht die Reinigungswirkung von erfindungsgemäßen (Kurve 1) und strukturell vergleichbaren, nicht erfindungsgemäßen (Kurve 2) Reinigungsmitteln, jeweils mit variierten Alkylenoxid-Gehalten, am Beispiel der Ölablösung von einer Metalloberfläche. Die Menge des von der Oberfläche der Metallplatte abgelösten Öles, ausgedrückt in % der ursprünglich aufgebrachten Menge, dient hier als Maß für die Reinigungswirkung der Reinigungsmittel.

Die in den Figuren 2a, 2b und 3 verwendeten Abkürzungen haben die folgende Bedeutung:

| | |
|---|---|
| AOC: | Gehalt des Mittels an Alkylenoxyd-Gruppen in mol/mol |
| DR: | Remissionsgrad in % der eingestrahlten Lichtmenge |
| EMPA 101 und wfk 10 D: | Bezeichnungen für angeschmutzte Gewebe |
| E40 und E60: | Kurven für Wäsche bei 40 bzw. 60°C mit erfindungsgemäßem Waschmitteln, |
| V40 und V60 | Kurven für Wäsche bei 40 bzw 60°C mit Vergleichsprodukt |
| OR | Ölablösung in % der im Anfang vorhandenen Ölmenge. |

Von erheblicher Bedeutung für die hohe Wirksamkeit der erfindungsgemäßen Wasch- und Reinigungsmittel ist, daß der in den Tensiden der Formel I enthaltene i - CₐH₂ₐ₊₁- Rest von einem Oxoalkohol abgeleitet ist, der durch Hydroformylierung eines Decens, Undecens Dodecens oder eines Gemisches dieser Olefine erhalten wird, das seinerseits durch Dimerisierung von n-Penten-2, Hexen-3 oder von Gemischen dieser Verbindungen hergestellt worden ist.

Der Mindestanteil der Tenside der Formel I am Gesamtgewicht der erfindungsgemäßen Wasch- und Reinigungsmittel wird so bemessen, daß sich eine signifikante Wirkung dieses Zusatzes zeigt.

Zweckmäßigerweise wird der Anteil der erfindungsgemäß einzusetzenden Tenside so eingestellt, daß sich im Zusammenwirken mit den übrigen Bestandteilen des Wasch- und Reinigungsmittels eine optimale Reinigungswirkung ergibt.

In der Regel wird eine gute Waschwirkung, insbesondere eine sehr gute Primärwaschwirkung der erfindungsgemäßen Waschmittel erreicht, wenn der Anteil der Tenside der Formel I in dem erfindungsgemäßen Waschmittel, bezogen auf das Gesamtgewicht des Mittels, 0,01 bis 50 Gew.-%, vorzugsweise 0,1 bis 40 Gew.-%, insbesondere 0,5 bis 30 Gew.-% beträgt.

Eine gute Reinigungswirkung der erfindungsgemäßen Reinigungsmittel wird in der Regel erreicht, wenn der Anteil der Tenside der Formel I in dem erfindungsgemäßen Reinigungsmittel, bezogen auf das Gesamtgewicht des Mittels, 0,01 bis 40 Gew.-%, vorzugsweise 0,1 bis 30 Gew.-%, insbesondere 0,1 bis 20 Gew.-% beträgt.

Bevorzugt sind Tenside der Formel I in denen a für den Wert 13 steht.

Vorteilhaft sind auch Tenside der Formel I, in denen n für eine Zahl von 1 bis 8, vorzugsweise von 1 bis 4 insbesondere für 1 oder 2 steht.
In einer weiteren bevorzugten Gruppe von Tensiden der Formel I sind R¹ und R² verschieden und bedeuten jeweils Wasserstoff, Methyl, Ethyl oder Propyl.

Ferner sind Tenside der Formel I bevorzugt, bei denen x für eine Zahl von 1 bis 50 insbesondere von 1 bis 20 und y für eine Zahl von 0 bis 50, insbesondere von 0 bis 20 steht.

Weiterhin sind Tenside der Formel I bevorzugt, bei denen die Summe von x und y im Bereich von 1 bis 200, vorzugsweise von 1 bis 100, insbesondere von 1 bis 50 liegt.

Bei einer weiteren speziellen Ausführungsform enthalten die erfindungsgemäßen Wasch- und Reinigungsmittel zumindest einen wirksamen Anteil von Verbindungen der Formel I, jedoch mit der Maßgabe, daß entweder die Summe von x und y mindestens 1 oder R³ ungleich Wasserstoff ist.

Die x Etherkettenglieder der Formel -CH₂-CHR¹- und die y Etherkettenglieder der Formel -CH₂-CHR²-können in den Verbindungen der Formel I blockweise oder statistisch in der Etherkette angeordnet sein.

Die Verbindungen der Formel I können einheitliche Substanzen sein, sie können aber auch Gemische darstellen, in denen verschiedene unter die Formel I fallenden Substanzen miteinander gemischt sind. Die Komponenten dieser Gemische können sich bezüglich der Bedeutungen von R¹, R² und auch R³, und hinsichtlich der Werte von a, und insbesondere von x und y unterscheiden. Dies hat zur Folge, daß die bei der Elementaranalyse der Tenside der Formel I erhaltenen analytischen Werte, beispielsweise die für die Oxoalkohol-Baugruppe erhaltenen C- und H-Werte, und insbesondere die Werte der Alkoxygruppen-Bestimmung bei der Rückrechnung auf die Strukturformel zu gebrochenen Werten für a, n, x und y führen. Selbstverständlich stellen auch derartige Substanzgemische unter die Formel I fallende erfindungsgemäße Tenside dar, die die beschriebenen Vorteile gegenüber dem Stand der Technik aufweisen.

Waschmittel im Sinne dieser Erfindung dienen in der Regel zum Waschen von mehr oder weniger flexiblen Materialien, vorzugsweise solchen, die natürliche, synthetische oder halbsynthetische Fasermaterialien enthalten oder daraus bestehen und die demzufolge in der Regel zumindest teilweise einen textilen Charakter aufweisen. Die faserhaltigen oder aus Fasern bestehenden Materialien können prinzipiell in jeder im Gebrauch oder der Herstellung und Verarbeitung vorkommenden Form vorliegen. Beispielsweise können Fasern ungeordnet in Form von Flocke oder Haufwerk, geordnet in Form von Fäden, Garnen, Zwirnen, oder in Form von Flächengebilden wie Vliesen, Lodenstoffen öder Filz, Geweben, Gewirken in allen denkbaren Bindungsarten vorliegen.
Es kann sich um Rohfasern oder um Fasern in beliebigen Verarbeitungsstadien handeln und es können natürliche Eiweiß- oder Zellulosefasern wie Wolle, Seide, Baumwolle, Sisal, Hanf, Kokosfasern oder Synthesefasern wie beispielsweise Polyester-, Polyamid- oder Polyacrylnitrilfasern sein.
Die erfindungsgemäßen Waschmittel können auch mit besonderem Vorteil im Rahmen der Verarbeitung von Fasermaterialien eingesetzt werden z.B. zum Entfetten von Rohwolle oder zum Entschlichten von Fasermaterialien aller Art.
Die erfindungsgemäßen Waschmittel können auch zur Reinigung von faserhaltigen Materialien, wie z.B. rückenbeschichteten Teppichen mit geschnittenem oder ungeschnittenem Flor dienen.

Das erfindungsgemäße Reinigungsmittel eignet sich besonders gut zur Reinigung von Materialien mit geschlossener, insbesondere harter Oberfläche, d.h. von Oberflächen, die keine oder nur wenige und kleine Poren haben und infolgedessen keine oder nur eine geringe Saugfähigkeit aufweisen. Materialien mit geschlossenen Oberflächen sind überwiegend hart, können aber auch weich sein in dem Sinne, daß sie eine gewisse reversible oder irreversible Verformbarkeit aufweisen.
Beispiele für Materialien mit harten Oberflächen, für deren Reinigung die erfindungsgemäßen Reinigungsmittel vorzugsweise eingesetzt werden, sind Metall, Glas, Email, Keramik. Typische Objekte aus diesen Materialien sind z.B. metallene Spülbecken, Bestecke, Glas- und Porzellangeschirr, Badewannen, Waschbecken, Kacheln, Fliesen und ausgehärtete Kunstharze, wie z.B. dekorative Melaminharzoberflächen auf Küchenmöbeln oder lackierte Metallflächen wie z.B. Kühlschränke und Autokarosserien. Die erfindungsgemäßen Reinigungsmittel sind auch sehr wertvolle Hilfsmittel bei der Herstellung von gedruckten Schaltungen, wobei es darum geht, Fettspuren und andere Verunreinigungen von kupfer- oder silberkaschierten Trägern vor dem Ätzen und/oder vor der Bestückung zu entfernen und/oder nach der Bestückung Lötfette oder andere Flußmittelreste gründlich zu beseitigen.
Auch bei der Herstellung von Mikrochips können die erfindungsgemäßen Reinigungsmittel wertvolle Dienste leisten. Materialien mit geschlossenen, insbesondere harten Oberflächen im Sinne dieser Erfindung können auch zerklüftete Oberflächen aufweisen, wie sie beispielsweise bei Metallkeramik-Werkstoffen zu finden sind.

Beispiele für weichere Materialien, die mit der erfindungsgemäßen Reinigungsmittel gereinigt werden können, sind beispielsweise versiegelte oder lackierte Hölzer, z.B. Parkett oder Wandverkleidungen, Fenstenahmen, Türen, Kunststoffbeläge wie Fußbodenbeläge aus PVC oder Hartgummi, oder Hart- oder Weichschaumstoffe mit weitgehend geschlossenen Oberflächen.
Insbesondere können die erfindungsgemäßen Reiniger beispielsweise als Handgeschirrreiniger, Maschinengeschirrreiniger, Metallentfetter, Glasreiniger, Fußbodenreiniger, Allzweckreiniger, Hochdruckreiniger, Neutralreiniger, alkalische Reiniger, saure Reiniger, Spritzentfetter, Molkereireiniger, Großküchenreiniger, Apparatereiniger in der Industrie, insbesondere der chemischen Industrie, als Reiniger bei der Autowäsche aber auch als Haushalts-Allzweckreiniger eingesetzt werden.

Selbstverständlich werden die Zusammensetzungen der Wasch- und Reinigungsmittel den verschiedenen Zwecken angepaßt, wie es dem Fachmann aus dem Stand der Technik geläufig ist. Hierzu können den erfindungsgemäßen Wasch- und Reinigungsmitteln alle zweckentsprechenden, aus dem oben genannten Stand der Technik bekannten Hilfs- und Zusatzstoffe zugefügt werden.

In vielen Fällen ist es zweckmäßig, die erfindungsgemäß eingesetzten Tenside der Formel I mit anderen nichtionischen Tensiden, wie z.B. Alkoholalkoxilaten, Alkylaminalkoxilaten, Alkylamidalkoxilaten, Alkylpolyglucosiden, oder mit ionischen, vorzugsweise anionischen, Tensiden, wie z.B. längerkettigen oder langkettigen Alkoholsulfat/ethersulfaten, Alkylbenzolsulfonaten, α-Olefinsulfonaten, Sulfosuccinaten, oder mit amphoteren Tensiden, wie z.B. Alkylaminoxiden, oder Betainen zu kombinieren.

Im Folgenden werden Beispiele für zur Kombination geeignete Tenside unterschiedlicher Natur genannt:

Als nichtionische Tenside eignen sich beispielsweise alkoxylierte C₈- bis C₂₂-Alkohole wie Fettalkoholalkoxylate oder Oxoalkoholalkoxylate. Die Alkoxylierung kann mit Ethylenoxid, Propylenoxid und/oder Butylenoxid durchgeführt werden. Als Tenside einsetzbar sind hierbei sämtliche alkoxylierten Alkohole, die vorzugsweise mindestens zwei Moleküle eines vorstehend genannten Alkylenoxids addiert enthalten. Auch hierbei kommen Blockpolymerisate von Ethylenoxid, Propylenoxid und/oder Butylenoxid in Betracht oder Anlagerungsprodukte, die die genannten Alkylenoxide in statistischer Verteilung enthalten. Pro Mol Alkohol verwendet man 2 bis 50, vorzugsweise 3 bis 20 Mol mindestens eines Alkylenoxids. Vorzugsweise setzt man als Alkylenoxid Ethylenoxid ein. Die Alkohole haben vorzugsweise 10 bis 18 Kohlenstoffatome. Je nach Art des Alkoxylierungskatalysators kann man Alkoxylate mit breiter oder enger Alkylenoxid-Homologen-Verteilung erhalten.

Eine weitere Klasse geeigneter nichtionischer Tenside sind Alkylphenolalkoxylate wie Alkylphenolethoxylate mit C₆ bis C₁₄-Alkylketten und 5 bis 30 Mol Alkylenoxideinheiten.

Eine andere Klasse nichtionischer Tenside sind Alkylpolyglucoside mit 6 bis 22, vorzugsweise 8 bis 18 Kohlenstoffatomen in der Alkylkette. Diese Verbindungen enthalten meist 1 bis 20, vorzugsweise 1,1 bis 5 Glucosideinheiten.

Eine andere Klasse nichtionischer Tenside sind N-Alkylglucamide der allgemeinen Strukturen 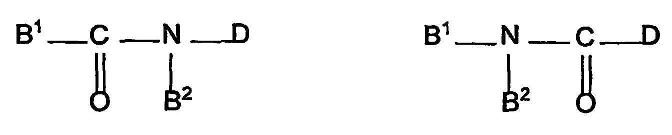 wobei B¹ ein C₆- bis C₂₂-Alkyl, B² Wasserstoff oder C₁- bis C₄-Alkyl und D ein Polyhydroxyalkyl-Rest mit 5 bis 12 C-Atomen und mindestens 3 Hydroxygruppen ist. Vorzugsweise steht B¹ für C₁₀- bis C₁₈-Alkyl, B² für CH₃ und D für einen C₅- oder C₆-Rest. Beispielsweise erhält man derartige Verbindungen durch die Acylierung von reduzierend aminierten Zuckern mit Säurechloriden von C₁₀- bis C₁₈-Carbonsäuren.

Weitere in Betracht kommende nichtionische Tenside sind die aus der WO-A 95/11225 bekannten endgruppenverschlossenen Fettsäureamidalkoxylate der allgemeinen Formel

R¹-CO-NH-(CH₂)_{y}-O-(A¹O)ₓ-R²

in der
- R¹: einen C₅- bis C₂₁-Alkyl- oder Alkenylrest bezeichnet,
- R²: eine C₁- bis C₄-Alkylgruppe bedeutet,
- A¹: für C₂- bis C₄-Alkylen steht,
- y: die Zahl 2 oder 3 bezeichnet und
- x: einen Wert von 1 bis 6 hat.

Beispiele für solche Verbindungen sind die Umsetzungsprodukte von n-Butyltriglykolamin der Formel H₂N-(CH₂-CH₂-O)₃-C₄H₉ mit Dodecansäuremethylester oder die Reaktionsprodukte von Ethyltetraglykolamin der Formel H₂N-(CH₂-CH₂-O)₄-C₂H₅ mit einem handelsüblichen Gemisch von gesättigten C₈- bis C₁₈-Fettsäuremethylestern.

Weiterhin eignen sich als nichtionische Tenside noch Blockcopolymere aus Ethylenoxid, Propylenoxid und/oder Butylenoxid (Pluronic®- und Tetronic®-Marken der BASF), Polyhydroxy- oder Polyalkoxyfettsäurederivate wie Polyhydroxyfettsäureamide, N-Alkoxy- oder N-Aryloxypolyhydroxyfettsäureamide, Fettsäureamidethoxylate, insbesondere endgruppenverschlossene, sowie Fettsäurealkanolamidalkoxylate.

Die zusätzlichen nichtionischen Tenside ("Niotenside") liegen in den erfindungsgemäßen Wasch- und Reinigungsmitteln vorzugsweise in einer Menge von 0,01 bis 30 Gew.-%, insbesondere 0,1 bis 25 Gew.-%, vor allem 0,5 bis 20 Gew.-%, vor.

Man kann einzelne nichtionische Tenside oder eine Kombination unterschiedlicher Niotenside einsetzen. Es können nichtionische Tenside aus nur einer Klasse zum Einsatz gelangen, insbesondere nur alkoxylierte C₈- bis C₂₂-Alkohole, man kann aber auch Tensidmischungen aus verschiedenen Klassen verwenden.

Geeignete anionische Tenside sind beispielsweise Fettalkoholsulfate von Fettalkoholen mit 8 bis 22, vorzugsweise 10 bis 18 Kohlenstoffatomen, z.B. C₉- bis C₁₁-Alkoholsulfate, C₁₂- bis C₁₄-Alkoholsulfate, C₁₂-C₁₈-Alkoholsulfate, Laurylsulfat, Cetylsulfat, Myristylsulfat, Palmitylsulfat, Stearylsulfat und Talgfettalkoholsulfat.

Weitere geeignete anionische Tenside sind sulfatierte ethoxylierte C₈- bis C₂₂-Alkohole (Alkylethersulfate) bzw. deren lösliche Salze. Verbindungen dieser Art werden beispielsweise dadurch hergestellt, daß man zunächst einen C₈- bis C₂₂-, vorzugsweise einen C₁₀- bis C₁₈-Alkohol z.B. einen Fettalkohol, alkoxyliert und das Alkoxylierungsprodukt anschließend sulfatiert. Für die Alkoxylierung verwendet man vorzugsweise Ethylenoxid, wobei man pro Mol Alkohol 1 bis 50, vorzugsweise 1 bis 20 Mol Ethylenoxid einsetzt. Die Alkoxylierung der Alkohole kann jedoch auch mit Propylenoxid allein und gegebenenfalls Butylenoxid durchgeführt werden. Geeignet sind außerdem solche alkoxylierte C₈- bis C₂₂-Alkohole, die Ethylenoxid und Propylenoxid oder Ethylenoxid und Butylenoxid oder Ethylenoxid und Propylenoxid und Butylenoxid enthalten. Die alkoxylierten C₈- bis C₂₂-Alkohole können die Ethylenoxid-, Propylenoxid- und Butylenoxideinheiten in Form von Blöcken oder in statistischer Verteilung enthalten. Je nach Art des Alkoxylierungskatalysators kann man Alkylethersulfate mit breiter oder enger Alkylenoxid-Homologen-Verteilung erhalten.

Weitere geeignete anionische Tenside sind Alkansulfonate wie C₈- bis C₂₄-, vorzugsweise C₁₀- bis C₁₈-Alkansulfonate sowie Seifen wie beispielsweise die Na- und K-Salze von C₈- bis C₂₄-Carbonsäuren.

Weitere geeignete anionische Tenside sind lineare C₈- bis C₂₀-Alkylbenzolsulfonate ("LAS"), vorzugsweise lineare C₉- bis C₁₃-Alkylbenzolsulfonate und -Alkyltoluolsulfonate.

Weiterhin eignen sich als anionische Tenside noch C₈- bis C₂₄-Olefinsulfonate und -disulfonate, welche auch Gemische aus Alken- und Hydroxyalkansulfonaten bzw. -disulfonate darstellen können, Alkylestersulfonate, sulfonierte Polycarbonsäuren, Alkylglycerinsulfonate, Fettsäureglycerinestersulfonate, Alkylphenolpolyglykolethersulfate, Paraffinsulfonate mit ca. 20 bis ca. 50 C-Atomen (basierend auf aus natürlichen Quellen gewonnenem Paraffin oder Paraffingemischen), Alkylphosphate, Acylisethionate, Acyltaurate, Acylmethyltaurate, Alkylbemsteinsäuren, Alkenylbernsteinsäuren oder deren Halbester oder Halbamide, Alkylsulfobernsteinsäuren oder deren Amide, Mono- und Diester von Sulfobernsteinsäuren, Acylsarkosinate, sulfatierte Alkylpolyglucoside, Alkylpolyglykolcarboxylate sowie Hydroxyalkylsarkosinate.

Die anionischen Tenside werden dem Wasch- und Reinigungsmittel vorzugsweise in Form von Salzen zugegeben. Geeignete Kationen in diesen Salzen sind Alkalimetallionen wie Natrium, Kalium und Lithium und Ammoniumsalze wie z.B. Hydroxyethylammonium-, Di(hydroxyethyl)ammonium- und Tri(hydroxyethyl)ammoniumsalze. Die anionischen Tenside liegen in den erfindungsgemäßen Waschmitteln vorzugsweise in einer Menge von bis zu 30 Gew.-%, beispielsweise von 0,1 bis 30 Gew.-%, vor allem 1 bis 25 Gew.%, insbesondere 3 bis 20 Gew.-% vor. Werden C₉- bis C₂₀-linear-Alkyl-benzolsulfonate (LAS) mitverwendet, kommen diese üblicherweise in einer Menge bis zu 15 Gew.-%, insbesondere bis zu 10 Gew.-%, zum Einsatz.
In den erfindungsgemäßen Reinigungsmitteln liegen die anionischen Tenside in einer Menge von bis zu 30 Gew.-%, vor allem bis zu 25 Gew.%, insbesondere bis zu 15 Gew.-% vor. Werden C₉- bis C₂₀-linear-Alkyl-benzolsulfonate (LAS) mitverwendet, kommen diese üblicherweise in einer Menge bis zu 10 Gew.-%, insbesondere bis zu 8 Gew.-%, zum Einsatz.

Man kann einzelne anionische Tenside oder eine Kombination unterschiedlicher Aniontenside einsetzen. Es können anionische Tenside aus nur einer Klasse zum Einsatz gelangen, beispielsweise nur Fettalkoholsulfate oder nur Alkylbenzolsulfonate, man kann aber auch Tensidmischungen aus verschiedenen Klassen verwenden,z.B. eine Mischung aus Fettalkoholsulfaten und Alkylbenzolsulfonaten.
Ferner können die erfindungsgemäß einzusetzenden Tenside der Formel I mit kationischen Tensiden, üblicherweise in einer Menge bis zu 25 Gew.-%, vorzugsweise 0,1 bis 15 Gew.-%, beispielsweise C₈-bis C₁₆-Dialkyldimethylammoniumhalogeniden, Dialkoxydimethylammoniumhalogeniden oder Imidazoliniumsalzen mit langkettigem Alkylrest; und/oder mit amphoteren Tensiden, üblicherweise in einer Menge bis zu 15 Gew.-%, vorzugsweise 0,1 bis 10 Gew.-%, beispielsweise Derivaten von sekundären oder tertiären Aminen wie z.B. C₆-C₁₈-Alkylbetainen oder C₆-C₁₅-Alkylsulfobetainen oder Aminoxiden wie Alkyldimethylaminoxiden kombiniert werden.

In der Regel werden die erfindunggemäß einzusetzenden Tenside der Formel I mit Buildern (Sequestrierungsmitteln) wie z.B. Polyphosphaten, Polycarboxilaten, Phosphonaten, Komplexbildnern, z.B. Methylglycindiessigsäure und deren Salze, Nitrilotriessigsäure und deren Salze, Ethylendiamintetraessigsäure und deren Salze sowie gegebenenfalls mit Co-Buildern kombiniert.

Einzelne zur Kombination mit den erfindungsgemäß einzusetzenden Tensiden der Formel I gut geeignete Buildersubstanzen seien im Folgenden aufgezählt:

Geeignete anorganische Builder sind vor allem kristalline oder amorphe Alumosilicate mit ionenaustauschenden Eigenschaften wie insbesondere Zeolithe. Verschiedene Typen von Zeolithen sind geeignet, insbesondere Zeolithe A, X, B, P, MAP und HS in ihrer Na-Form oder in Formen, in denen Na teilweise gegen andere Kationen wie Li, K, Ca, Mg oder Ammonium ausgetauscht ist. Geeignete Zeolithe sind beispielsweise beschrieben in der US-A-4604224.

Als Builder geeignete kristalline Silicate sind beispielsweise Disilicate oder Schichtsilicate, z.B. δ-Na₂Si₂O₅ oder β-Na₂Si₂O₅ (SKS 6 bzw. SKS 7). Die Silicate können in Form ihrer Alkalimetall-, Erdalkalimetall- oder Ammoniumsalze eingesetzt werden, vorzugsweise als Na-, Li- und Mg-Silicate.Amorphe Silicate wie beispielsweise Natriummetasilicat, welches eine polymere Struktur aufweist, oder amorphes Disilicat (Britesil® H 20 Hersteller: Akzo) sind ebenfalls verwendbar.

Geeignete anorganische Buildersubstanzen auf Carbonat-Basis sind Carbonate und Hydrogencarbonate. Diese können in Form ihrer Alkalimetall-, Erdalkalimetall- oder Ammoniumsalze eingesetzt werden. Vorzugsweise werden Na-, Li- und Mg-Carbonate bzw. -Hydrogencarbonate, insbesondere Natriumcarbonat und/oder Natriumhydrogencarbonat, eingesetzt. Übliche, als anorganische Builder eingesetzte Phosphate sind Alkali-orthophosphate,und/oder -Polyphosphate wie z.B. Pentanatriumtriphosphat. Die genannten Builder-Komponenten können einzeln oder in Mischungen untereinander eingesetzt werden.

Ferner ist es in vielen Fällen zweckmäßig den erfindungsgemäßen Wasch- und Reinigungsmitteln Co-Builder zuzufügen. Beispiele für geeignete Substanzen sind im Folgenden aufgelistet:

In einer bevorzugten Ausführungsform enthalten die erfindungsgemäßen Wasch- und Reinigungsmittel zusätzlich zu den anorganischen Buildern 0,05 bis 20 Gew.-%, insbesondere 1 bis 10 Gew.-% organische Cobuilder in Form von niedermolekularen, oligomeren oder polymeren Carbonsäuren, insbesondere Polycarbonsäuren, oder Phosphonsäuren oder deren Salzen, insbesondere Na- oder K-salzen.

Als organische Cobuilder geeignete niedermolekulare Carbonsäuren oder Phosphonsäuren sind beispielsweise:

Phosphonsäuren wie z.B. 1-Hydroxyethan-1,1-diphosphonsäure, Amino-tris(methylenphosphonsäure), Ethylendiamin-tetra(methylenphosphonsäure), Hexamethylendiamin-tetra (methylenphosphonsäure) und Diethylentriamin-penta(methylenphosphonsäure); C₄-bis C₂₀-Di-, -Tri- und -Tetracarbonsäuren wie z.B. Bernsteinsäure, Propantricarbonsäure, Butantetracarbonsäure, Cyclopentantetracarbonsäure und Alkyl- und Alkenylbernsteinsäuren mit C₂- bis C₁₆-Alkyl- bzw. -Alkenyl-Resten; C₄- bis C₂₀-Hydroxycarbonsäuren wie z.B. Äpfelsäure, Weinsäure, Gluconsäure, Glutarsäure, Citronensäure, Lactobionsäure und Saccharosemono-, -di- und -tricarbonsäure; Aminopolycarbonsäuren wie z.B. Nitrilotriessigsäure, β-Alanindiessigsäure, Ethylendiamintetraessigsäure, Serindiessigsäure, Isoserindiessigsäure, Alkylethylendiamintriacetate, N,N-bis(Carboxymethyl)glutaminsäure, Ethylendiamindibernsteinsäure und N-(2-Hydroxyethyl)iminodiessigsäure, Methyl- und Ethylglycindiessigsäure.

Als organische Cobuilder geeignete oligomere oder polymere Carbonsäuren sind beispielsweise:

Oligomaleinsäuren, wie sie beispielsweise in EP-A 451508 und EP-A 396303 beschrieben sind; Co- und Terpolymere ungesättigter C₄- bis C₈-Dicarbonsäuren, wobei als Comonomere monoethylenisch ungesättigte Monomere aus der unten angegebenen Gruppe (i) in Mengen von bis zu 95 Gew.-%, aus der Gruppe (ii) in Mengen von bis zu 60 Gew.-% und aus der Gruppe (iii) in Mengen von bis zu 20 Gew.-% einpolymerisiert sein können.

Als ungesättigte C₄- bis C₈-Dicarbansäuren sind hierbei beispielsweise Maleinsäure, Fumarsäure, Itaconsäure und Citraconsäure geeignet. Bevorzugt wird Maleinsäure.

Die Gruppe (i) umfaßt monoethylenisch ungesättigte C₃-C₈-Monocarbonsäuren wie z.B. Acrylsäure, Methacrylsäure, Crotonsäure und vinylessigsäure. Bevorzugt werden aus der Gruppe (i) Acrylsäure und Methacrylsäure eingesetzt.

Die Gruppe (ii) umfaßt monoethylenisch ungesättigte C₂- bis C₂₂-Olefine, Vinylalkylether mit C₁- bis C₈-Alkylgruppen, Styrol, Vinylester von C₁- bis C₈-Carbonsäuren, (Meth)acrylamid und Vinylpyrrolidon. Bevorzugt werden aus der Gruppe (ii) C₂- bis C₆-Olefine, Vinylalkylether mit C₁- bis C₄-Alkylgruppen, Vinylacetat und Vinylpropionat eingesetzt.

Falls die Polymeren der Gruppe (ii) Vinylester einpolymerisiert enthalten, können diese auch teilweise oder vollständig zu Vinylalkohol-Struktureinheiten hydrolysiert vorliegen. Geeignete Co- und Terpolymere sind beispielsweise aus US-A 3887806 sowie DE-A 4313909 bekannt.

Die Gruppe (iii) umfaßt (Meth)acrylester von C₁- bis C₈-Alkoholen, (Meth)acrylnitril, (Meth)acrylamide von C₁- bis C₈-Aminen, N-Vinylformamid und N-Vinylimidazol.

Als organische Cobuilder eignen sich auch Homopolymere der monoethylenisch ungesättigten C₃-C₈-Monocarbonsäuren wie z.B. Acrylsäure, Methacrylsäure, Crotonsäure und Vinylessigsäure, insbesondere der Acrylsäure und Methacrylsäure, Copolymere von Dicarbonsäuren, wie z.B. Copolymere von Maleinsäure und Acrylsäure im Gewichtsverhältnis 10:90 bis 95:5, besonders bevorzugt solche im Gewichtsverhältnis 30:70 bis 90:10 mit Molmassen von 1000 bis 150000;

Terpolymere aus Maleinsäure, Acrylsäure und einem Vinylester einer C₁-C₃-Carbonsäure im Gewichtsverhältnis 10 (Maleinsäure) :90 (Acrylsäure + Vinylester) bis 95 (Maleinsäure) :10 (Acrylsäure + Vinylester), wobei das Gew.-Verhältnis von Acrylsäure zum Vinylester im Bereich von 30:70 bis 70:30 variieren kann;

Copolymere von Maleinsäure mit C₂-C₈-Olefinen im Molverhältnis 40:60 bis 80:20, wobei Copolymere von Maleinsäure mit Ethylen, Propylen oder Isobuten im Molverhältnis 50:50 besonders bevorzugt sind.

Pfropfpolymere ungesättigter Carbonsäuren auf niedermolekulare Kohlenhydrate oder hydrierte Kohlenhydrate, vgl. US-A 5227446, DE-A 4415623 und DE-A 4313909, eignen sich ebenfalls als organische Cobuilder.

Geeignete ungesättigte Carbonsäuren sind hierbei beispielsweise Maleinsäure, Fumarsäure, Itaconsäure, Citraconsäure, Acrylsäure, Methacrylsäure, Crotonsäure und Vinylessigsäure sowie Mischungen aus Acrylsäure und Maleinsäure, die in Mengen von 40 bis 95 Gew.-%, bezogen auf die zu pfropfende Komponente, aufgepfropft werden.

Zur Modifizierung können zusätzlich bis zu 30 Gew.-%, bezogen auf die zu pfropfende Komponente, weitere monoethylenisch ungesättigte Monomere einpolymerisiert vorliegen. Geeignete modifizierende Monomere sind die oben genannten Monomere der Gruppen (ii) und (iii).

Als Pfropfgrundlage sind abgebaute Polysaccharide wie z.B. sauer oder enzymatisch abgebaute Stärken, Inuline oder Zellulose, Eiweißhydrolysate und reduzierte (hydrierte oder hydrierend aminierte) abgebaute Polysaccharide wie z.B. Mannit, Sorbit, Aminosorbit und N-Alkylglucamin geeignet sowie auch Polyalkylenglycole mit Molmassen mit bis zu M_{w} = 5000 wie z. B. Polyethylenglycole, Ethylenoxid/Propylenoxid- bzw. Ethylenoxid/Butylenoxid bzw. Ethylenoxid/Propylenoxid/Butylenoxid-Blockcopolymere und alkoxylierte ein- oder mehrwertige C₁- bis C₂₂-Alkohole.(vgl. US-A-5756456)

Als organische Cobuilder geeignete Polyglyoxylsäuren sind beispielsweise beschrieben in EP-B-001004, US-A-5399286, DE-A-4106355 und EP-A-656914. Die Endgruppen der Polyglyoxylsäuren können unterschiedliche Strukturen aufweisen.

Als organische Cobuilder geeignete Polyamidocarbonsäuren und modifizierte Polyamidocarbonsäuren sind beispielsweise bekannt aus EP-A-454126, EP-B-511037, WO-A-94/01486 und EP-A-581452.

Als organische Cobuilder verwendet man insbesondere auch Polyasparaginsäuren oder Cokondensate der Asparaginsäure mit weiteren Aminosäuren, C₄- bis C₂₅-Mono- oder -Dicarbonsäuren und/oder C₄- bis C₂₅-Mono- oder -Diaminen. Besonders bevorzugt werden in phosphorhaltigen Säuren hergestellte, mit C₆- bis C₂₂-Mono- oder -Dicarbonsäuren bzw. mit C₆- bis C₂₂-Mono- oder -Diaminen modifizierte Polyasparaginsäuren eingesetzt.

Als organische Cobuilder eignen sich weiterhin Iminodibernsteinsäure, Oxydibernsteinsäure, Aminopolycarboxylate, Alkylpolyaminocarboxylate, Aminopolyalkylenphosphonate, Polyglutamate, hydrophob modifizierte Citronensäure wie z.B. Agaricinsäure, Poly-α-hydroxyacrylsäure, N-Acylethylendiamintriacetate wie Lauroylethylendiamintriacetat und Alkylamide der Emylendiamintetraessigsäure wie EDTA-Talgamid.

Weiterhin können auch oxidierte Stärken als organische Cobuilder verwendet werden.

In einer weiteren bevorzugten Ausführungsform enthalten die erfindungsgemäßen Wasch- und Reinigungsmittel zusätzlich, insbesondere zusätzlich zu den anorganischen Buildern, den anionischen Tensiden und/oder den nichtionischen Tensiden, 0,5 bis 20 Gew.-%, insbesondere 1 bis 10 Gew.-%, Glycin-N,N-diessigsäure-Derivate, wie sie in der WO 97/19159 beschrieben sind.

Häufig ist es auch zweckmäßig, den erfindungsgemäßen Wasch- und Reinigungsmitteln Bleichsysteme, bestehend aus Bleichmitteln, wie z.B. Perborat, Percarbonat und gegebenenfalls Bleichaktivatoren, wie z.B. Tetraacetylethylendiamin, + Bleichstabilisatoren zuzusetzen.

In diesen Fällen enthalten die erfindungsgemäßen Wasch- und Reinigungsmittel zusätzlich 0,5 bis 30 Gew.-%, insbesondere 5 bis 27 Gew.-%, vor allem 10 bis 23 Gew.-% Bleichmittel in Form von Percarbonsäuren, z.B. Diper-oxododecandicarbonsäure, Phthalimidopercapronsäure oder Monoperoxophthalsäure oder -terephthalsäure, Addukten von Wasserstoffperoxid an anorganische Salze, z.B. Natriumperborat-Monohydrat, Natriumperborat-Tetrahydrat, Namumcarbonat-Perhydrat oder Natriumphosphat-Perhydrat, Addukten von Wasserstoffperoxid an organische Verbindungen, z.B. Harnstoff-Perhydrat, oder von anorganischen Peroxosalzen, z.B. Alkalimetallpersulfaten, oder - peroxodisulfaten, gegebenenfalls in Kombination mit 0 bis 15 Gew.-%, vorzugsweise 0,1 bis 15 Gew.-%, insbesondere 0,5 bis 8 Gew.-%, Bleichaktivatoren.

Als Bleichaktivatoren eignen sich:
- polyacylierte Zucker, z.B. Pentaacetylglucose;
- Acyloxybenzolsulfonsäuren und deren Alkali- und Erdalkaümetallsalze, z.B. Natrium-p-nonanoyloxybenzolsulfonat oder Natrium-p-benzoyloxybenzolsulfonat;
- N,N-diacylierte und N,N,N',N'-tetraacylierte Amine, z.B. N,N,N',N'-Tetraacetylmethylendiamin und -ethylendiamin (TAED), N,N-Diacetylanilin, N,N-Diacetyl-p-toluidin oder 1,3-diacylierte Hydantoine wie 1,3-Diacetyl-5,5-dimethylhydantoin;
- N-Alkyl-N-sulfonylcarbonamide, z.B. N-Methyl-N-mesylacetamid oder N-Methyl-N-mesylbenzamid;
- N-acylierte cyclische Hydrazide, acylierte Triazole oder Urazole, z.B. Monoacetylmaleinsäurehydrazid;
- O,N,N-trisubstituierte Hydroxylamine, z.B. O-Benzoyl-N,N-succinylhydroxylamin, O-Acetyl-N,N-succinylhydroxylamin oder O,N,N-Triacetylhydroxylamin;
- N,N'-Diacylsulfurylamide, z.B. N,N'-Dimethyl-N,N'-diacetylsulfurylamid oder N,N'-Diethyl-N,N'-dipropionylsulfinylamid;
- acylierte Lactame wie beispielsweise Acetylcaprolactam, Octanoylcaprolactam, Benzoylcaprolactam oder Carbonylbiscaprolactam;
- Anthranilderivate wie z.B. 2-Methylanthranil oder 2-Phenylanthranil;
- Triacylcyanurate, z.B. Triacetylcyanurat oder Tribenzoylcyanurat;
- Oximester und Bisoximester wie z.B. O-Acetylacetonoxim oder Bisisopropyliminocarbonat;
- Carbonsäureanhydride, z.B. Essigsäureanhydrid, Benzoesäureanhydrid, m-Chlorbenzoesäureanhydrid oder Phthalsäureanhydrid;
- Enolester wie z.B. Isopropenylacetat;
- 1,3-Diacyl-4,5-diacyloxy-imidazoline, z.B. 1,3-Diacetyl-4,5-diacetoxyimidazolin;
- Tetraacetylglycoluril und Tetrapropionylglycoluril; diacylierte 2,5-Diketopiperazine, z.B. 1,4-Diacetyl-2,5-diketopiperazin;
- ammoniumsubstituierte Nitrile wie z.B. N-Methylmorpholiniumacetonitrilmethylsulfat;
- Acylierungsprodukte von Propylendihamstoff und 2,2-Dimethylpropylendiharnstoff, z.B. Tetraacetylpropylendiharnstoff;
- α-Acyloxypolyacylmalonamide, z.B. α-Acetoxy-N,N'-diacetylmalonamid;
- Diacyl-dioxohexahydro-1,3,5-triazine, z.B. 1,5-Diacetyl-2,4-dioxohexahydro-1,3,5-triazin;
- Benz-(4H)1,3-oxazin-4-one mit Alkylresten, z.B. Methyl, oder aromatischen Resten z.B. Phenyl, in der 2-Position.

Das beschriebene Bleichsystem aus Bleichmitteln und Bleichaktivatoren kann gegebenenfalls noch Bleichkatalysatoren enthalten. Geeignete Bleichkatalysatoren sind beispielsweise quaternierte Imine und Sulfonimine, die beispielsweise beschrieben sind in US-A 5 360 569 und EP-A 453 003. Besonders wirksame Bleichkatalysatoren sind Mangankomplexe, die beispielsweise in der WO-A 94/21777 beschrieben sind. Solche Verbindungen werden im Falle ihres Einsatzes in den Wasch- und Reinigungsmitteln höchstens in Mengen bis 1,5 Gew.-%, insbesondere bis 0,5 Gew.-%, im Falle von sehr aktiven Mangankomplexen in Mengen bis zu 0,1 Gew.-%, eingearbeitet.

Neben dem beschriebenen Bleichsystem aus Bleichmitteln, Bleichaktivatoren und gegebenenfalls Bleichkatalysatoren ist für die erfindungsgemäßen Wasch- und Reinigungsmittel auch die Verwendung von Systemen mit enzymatischer Peroxidfreisetzung oder von photoaktivierten Bleichsystemen möglich.

Für eine Reihe von Anwendungsfällen ist es zweckmäßig, wenn die erfindungsgemäßen Wasch- und Reinigungsmittel Enzyme enthalten. Vorzugsweise in Wasch- und Reinigungsmitteln eingesetzte Enzyme sind Proteasen, Amylasen, Lipasen und Cellulasen. Von den Enzymen werden vorzugsweise mengen von 0,1 bis 1,5 Gew.-%, insbesondere vorzugsweise 0,2 bis 1,0 Gew.-%, des konfektionierten Enzyms zugesetzt. Geeignete Proteasen sind z.B. Savinase und Esperase (Hersteller. Novo Nordisk). Eine geeignete Lipase ist z. B. Lipolase (Hersteller: Novo Nordisk). Eine geeignete Cellulase ist z.B. Celluzym (Hersteller: Novo Nordisk). Auch die Verwendung von Peroxidasen zur Aktivierung des Bleichsystems ist möglich. Man kann einzelne Enzyme oder eine Kombination unterschiedlicher Enzyme einsetzen. Gegebenenfalls kann das erfindungsgemäße Wasch- und Reinigungsmittel noch Enzymstabilisatoren, z.B. Calciumpropionat, Natriumformiat oder Borsäuren oder deren Salze, und/oder Oxidationsverhinderer enthalten.

Die Bestandteile von Wasch- und Reinigungsmitteln sind dem Fachmann prinzipiell bekannt. Die obigen und die weiter unten folgenden Listen geeigneter Bestandteile geben nur einen exemplarischen Ausschnitt der bekannten geeigneten Bestandteile wieder.

Die erfindungsgemäßen Wasch- und Reinigungsmittel können neben den bisher genannten Hauptkomponenten noch folgende weitere übliche Zusätze in den hierfür üblichen Mengen enthalten:

Bekannte Dispergiermittel, wie z.B. Naphthalinsulfonsäurekondensate oder Polycarboxilate, pH-regulierende Verbindungen wie z.B. Alkalien bzw. Alkalispender (NaOH, KOH, Pentanatriummetasilikat) oder Säuren (Salzsäure, Phosphorsäure, Amidoschwefelsäure, Citronensäure) Puffersysteme, wie z.B. Acetat oder Phosphatpuffer, Parfüm, Farbstoffe, Biozide, wie z.B. Isothiazolinone oder 2-Bromo-2-nitro-1,3-propandiol,

Solubilisatoren/Hydrotrope, wie z.B. Cumolsulfonate, Toluolsulfonate, kurzkettige Fettsäuren, Harnstoff, Alkohole oder Phosphorsäurealkyl/-arylester, Alkyl/Arylpolyglycolphosphorsäureester, Schaumregulatoren zur Stabilisierung oder Dämpfung des Schaums, Haut- und Korrosionsschutzmittel, desinfizierende Verbindungen oder Systeme, wie z.B. solche die Chlor oder unterchlorige Säure freisetzen wie z.B. Dichlorisocyanurat oder die Jod enthalten.

Die Waschmittel enthalten gegebenenfalls zusätzlich Schmutztragemittel, Soil release Agentien, wie z.B. Polyetherester, Inkrustationsinhibitoren, Ionenaustauscher, Vergrauungsinhibitoren, optische (fluoreszierende) Aufheller, Farbübertragungsinhibitoren wie z.B. Polyvinylpyrrolidon, Verdickungsmittel und Stell- und Konfektionierungsmittel, in Reinigungsmitteln können zusätzlich Lösemittel, wie z.B. kurzkettige Alkyloligoglykole wie Butylglykol, Butyldiglykol, Propylenglykolmonomethylether, Alkohole wie Ethanol, i-Propanol, aromatische Lösemittel wie Toluol, Xylol, N-Alkylpyrrolidone oder Alkylencarbonate, Verdicker, wie z.B. Polysaccharide, und/oder schwach vernetzte Polycarboxylate (beispielsweise Carbopol® der Firma Goodrich) feinteilige Abrasivkomponenten, wie z.B.Quarz- oder Marmormehl, Kreide, Diatomeenerde, Bimsstein oder auch Polierrot oder Schmirgel, enthalten sein.

Die Waschmittel liegen gewöhnlich, aber nicht ausschließlich, in fester, pulverförmiger Form vor, und enthalten dann in der Regel zusätzlich übliche Stellmittel, die ihnen eine gute Rieselfähigkeit, Dosierbarkeit und Löslichkeit verleihen und die das Zusammenbacken und Stauben verhüten, wie z.B. Natriumsulfat oder Magnesiumsulfat. Die pulverförmigen Waschmittel haben in der herkömmlichen Form ein durchschnittliches Schüttgewicht von ca. 450 g/l. Kompakt- oder Ultra-Kompaktwaschmittel sowie Extrudate weisen ein Schüttgewicht > 600 g/l auf Diese gewinnen immer mehr an Bedeutung.

Sofern sie in flüssiger Form eingesetzt werden sollen, können sie als wäßrige Mikroemulsionen, Emulsionen oder Lösungen vorliegen. In flüssigen Waschmitteln können zusätzlich Lösungsmittel wie z.B. Ethanol, i-Propanol, 1,2-Propylenglykol, oder Butylglykol verwendet werden.

Bei gelförmigen erfindungsgemäßen Waschmitteln können zusätzlich Verdicker, wie z.B. Polysaccharide und/oder schwach vernetzte Polycarboxylate (beispielsweise Carbopol® der Fa. Goodrich) eingesetzt werden.

Bei tablettenförmigen Waschmitteln werden zusätzlich Tablettierhilfsmittel wie z.B. Polyethylenglykole mit Molmassen > 1000 g/mol, Polymerdispersionen, und Tablettensprengmittel wie z.B. Cellulosederivate, vernetztes Polyvinylpyrrolidon, vernetzte Polyacrylate oder Kombinationen aus Säuren, z.B. Citronensäure + Natriumbicarbonat, um nur einige zu nennen, benötigt.

Die erfindungsgemäßen Waschmittel sind in Ihrer Waschwirkung nächstvergleichbaren Waschmitteln überraschenderweise erheblich überlegen. Wie der im Rahmen der Ausführungsbeispiele durchgeführte Vergleich zeigt, weisen sie gegenüber den bekannten C₁₃-Oxoalkohol-Tensiden, die auf der Basis von trimerisierten Butenen hergestellt wurden, deutliche Vorteile insbesondere in der Primärwaschwirkung auf.

Die Reinigungsmittel sind gewöhnlich, aber nicht ausschließlich, wäßrig und liegen in der Form von Milaoemulsionen, Emulsionen oder Lösungen vor.

Sollten sie in fester, pulverförmiger Form vorliegen, können zusätzlich übliche Stellmittel, die ihnen eine gute Rieselfähigkeit, Dosierbarkeit und Löslichkeit verleihen und/oder die das Zusammenbacken und Stauben verhüten, wie z.B. Natriumsulfat oder Magnesiumsulfat eingesetzt werden.

Bei tablettenförmigen Reinigern werden zusätzlich Tablettierhilfsmittel wie z.B. Polyethylenglykole mit Molmassen > 1000 g/mol, Polymerdispersionen, und Tablettensprengmittel wie z.B. Cellulosederivate, vernetztes Polyvinylpyrrolidon, vernetzte Polyacrylate oder Kombinationen aus Säuren, z.B. Citronensäure + Natriumbicarbonat, um nur einige zu nennen, benötigt.

Die erfindungsgemäßen Wasch- und Reinigungsmittel sind in Ihrer Reinigungswirkung nächstvergleichbaren Wasch- und Reinigungsmitteln überraschenderweise erheblich überlegen. Wie der im Rahmen der Ausführungsbeispiele durchgeführte Vergleich zeigt, weisen sie gegenüber den bekannten C₁₃-Oxoalkohol-Tensiden, die auf der Basis von trimerisierten Butenen hergestellt wurden, deutliche Vorteile in der Reinigungsleistung auf.

Ein weiterer Gegenstand der vorliegenden Erfindung ist ein Verfahren zur Herstellung von Wasch- und Reinigungsmitteln, bei welchem Tenside der Formel I eingesetzt werden.

Weitere Gegenstände der Erfindung sind ein Waschverfahren unter Einsatz eines erfindungsgemäßen Waschmittels und die Verwendung eines erfindungsgemäßen Reinigungsmittels zur Reinigung von geschlossenen, vorzugsweise harten Oberflächen.

Von ausschlaggebenden Bedeutung für die überraschend hohe Wirksamkeit der erfindungsgemäß eingesetzten Tenside der Formel I gegenüber nächstvergleichbaren Verbindungen ist offenbar die Struktur des Restes CₐH₂ₐ₊₁O-.

Diese überraschend wirksame Struktur der erfindungsgemäß einzusetzenden Tenside wird erhalten, wenn sie dadurch hergestellt werden, daß man
a) ein C₄-Olefin-Gemisch der Metathese unterwirft,
b) aus dem Metathesegemisch Olefine mit 5 und 6 C-Atomen abtrennt,
c) die abgetrennten Olefine einzeln oder im Gemisch einer Dimerisierung zu Olefingemischen mit 10 bis 12 C-Atomen unterzieht,
d) das erhaltene Olefingemisch, gegebenenfalls nach einer Fraktionierung, der Derivatisierung zu einem Gemisch von Oxoalkoholen(Tensidalkoholen) unterwirft und
e) gegebenenfalls die erhaltenen Oxoalkohole alkoxyliert,
f) gegebenenfalls die in den Herstellungsschritten d) oder e) erhaltenen Oxoalkohole bzw Oxoalkohol-alkoxylate in die sauren Schwefelsäure- oder Phosphorsäureester überführt.

Die Figur 1 veranschaulicht diesen Syntheseweg zu den erfindungsgemäß einzusetzenden Tensiden der Formel I

Die Grundzüge der im Verfahrensschritt a) eingesetzten Metathese sind beispielsweise in Ullmann's Encyclopedia of Industrial Chemistry, 5. Aufl., Band A18, S.235/236 beschrieben worden. Weitere Informationen zur Durchführung des Verfahrens können beispielsweise K.J. Ivin, "Olefin Metathesis", Academic Press, London, (1983); Houben-Weyl, **E18**, 1163-1223; R.L. Banks, Discovery and Development of Olefin Disproportionation, CHEMTECH (**1986**), February, 112-117, entnommen werden.

Bei der Anwendung der Metathese auf die in den C₄-Olefin-Strömen enthaltenen Hauptbestandteile Buten-1 und Buten-2 werden in Gegenwart von geeigneten Katalysatoren Olefine mit 5 bis 10 C-Atomen, vorzugsweise mit 5 bis 8 C-Atomen, insbesondere aber Penten-2 und Hexen-3 gebildet.

Geeignete Katalysatoren sind vorzugsweise Molybdän-, Wolfram- oder Rheniumverbindungen. Es ist besonders zweckmäßig, die Reaktion heterogenkatalysiert auszuführen, wobei die katalytisch wirksamen Metalle insbesondere in Verbindung mit Trägern aus Al₂O₃ oder SiO₂ eingesetzt werden. Beispiele für derartige Katalysatoren sind MoO₃ oder WO₃ auf SiO₂, oder Re₂O₇ auf Al₂O₃.

Besonders günstig ist es, die Metathese in Gegenwart eines Rheniumkatalysators auszuführen, da in diesem Fall besonders milde Reaktionsbedingungen möglich sind. So kann die Metathese in diesem Fall bei einer Temperatur von 0 bis 50°C und bei niedrigen Drucken von ca. 0,1 bis 0,2 MPa ausgeführt werden.

Bei der Dimerisierung der im Metatheseschritt erhaltenen Olefine oder Olefingemische erhält man Dimerisienmgsprodukte, die im Hinblick auf die weitere Verarbeitung auf Tensidalkohole besonders günstige Komponenten und eine besonders vorteilhafte Zusammensetzung aufweisen, wenn man einen Dimerisierungskatalysator einsetzt, der wenigstens ein Element der VIII. Nebengruppe des periodischen Systems enthält.

Ferner ist es vorteilhaft, die Katalysatorzusammensetzung und die Reaktionsbedingungen so zu wählen, daß ein Dimerengemisch erhalten wird, welches weniger als 10 Gew.-% von Verbindungen enthält, die ein Strukturelement der Formel I (Vinylidengruppe) 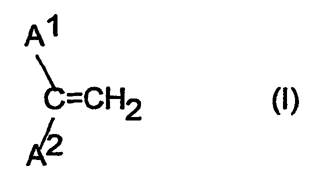 worin A¹ und A² aliphatische Kohlenwasserstoffreste sind,
aufweisen.

Vorzugsweise werden für die Dimerisierung die in dem Metathesierungsprodukt enthaltenen internen, linearen Pentene und Hexene eingesetzt. Besonders bevorzugt ist der Einsatz von 3-Hexen.

Die Dimerisierung kann homogenkatalysiert oder heterogenkatalysiert durchgeführt werden. Bevorzugt ist die heterogene Verfahrensweise, da hierbei einerseits die Katalysatorabtrennung vereinfacht und das Verfahren damit wirtschaftlicher ist, zum anderen werden keine umweltschädlichen Abwässer erzeugt, wie sie gewöhnlich bei der Abtrennung gelöster Katalysatoren, zum Beispiel durch Hydrolyse, anfallen. Ein weiterer Vorteil des heterogenen Verfahrens besteht darin, daß das Dimerisierungsprodukt keine Halogene, insbesondere Chlor oder Fluor, enthält. Homogen lösliche Katalysatoren enthalten im allgemeinen halogenidhaltige Liganden oder sie werden in Kombination mit halogenhaltigen Cokatalysatoren eingesetzt. Aus solchen Katalysatorsystemen kann Halogen in die Dimerisierungsprodukte eingebaut werden, was sowohl die Produktqualität als auch die Weiterverarbeitung, insbesondere die Hydroformylierung zu Tensidalkoholen erheblich beeinträchtigt.

Zur heterogenen Katalyse werden zweckmäßigerweise Kombinationen von Oxiden von Metallen der VIII. Nebengruppe mit Aluminiumoxid auf Trägermaterialien aus Silizium- und Titanoxiden wie sie beispielsweise aus der DE-A-43 39 713 bekannt sind, eingesetzt. Der heterogene Katalysator kann im Festbett - dann vorzugsweise in grobkörniger Form als 1 bis 1,5 mm-Splitt - oder suspendiert (Partikelgröße 0.05 bis 0,5 mm) eingesetzt werden. Die Dimerisierung wird bei heterogener Durchführung zweckmäßigerweise bei Temperaturen von 80 bis 200°C, vorzugsweise von 100 bis 180°C, unter dem bei der Reaktionstemperatur herrschenden Druck, gegebenenfalls auch unter einem Schutzgasüberdruck, im geschlossenen System ausgeführt. Zur Erzielung optimaler Umsätze wird das Reaktionsgemisch mehrfach im Kreis geführt, wobei kontinuierlich ein bestimmter Anteil des zirkulierenden Produkts ausgeschleust und durch Ausgangsmaterial ersetzt wird.

Bei der Dimerisierung werden Mischungen einfach ungesättigter Kohlenwasserstoffe erhalten, deren Komponenten überwiegend die doppelte Kettenlänge haben wie die Ausgangs-Olefine.

Die Dimerisierungskatalysatoren und die Reaktionsbedingungen werden im Rahmen der obigen Angaben zweckmäßigerweise so gewählt, daß mindestens 80 % der Komponenten des Dimerisierungsgemisches im Bereich von 1/4 bis 3/4, vorzugsweise von 1/3 bis 2/3, der Kettenlänge ihrer Hauptkette eine Verzweigung, oder zwei Verzweigungen an benachbarten C-Atomen, aufweisen.

Sehr charakteristisch für die so hergestellten Olefingemische ist ihr hoher Anteil - in der Regel über 75%, insbesondere über 80% - von Komponenten mit Verzweigungen und der geringe Anteil - in der Regel unter 25, insbesondere unter 20% - unverzweigter Olefine. Ein weiteres Charakteristikum ist, daß an den Verzweigungsstellen der Hauptkette überwiegend Gruppen mit (y-4) und (y-5) C-Atomen gebunden sind, wobei y die Kohlenstoffatom-Anzahl des für die Dimerisierung eingesetzten Monomers ist. Der Wert (y-5)=0 bedeutet, daß keine Seitenkette vorhanden ist.

Bei den so hergestellten C₁₂-Olefingemischen trägt die Hauptkette an den Verzweigungspunkten vorzugsweise Methyl- oder Ethylgruppen.

Die Stellung der Methyl- und Ethylgruppen an der Hauptkette ist ebenfalls charakteristisch: Bei Monosubstitution befinden sich die Methyl- oder Ethylgruppen in der Position P = (n/2)-m der Hauptkette, wobei n die Länge der Hauptkette und m die Kohlenstoffanzahl der Seitengruppen ist, bei Disubstitutionsprodukten befindet sich ein Substituent in der Position P, der andere am benachbarten C-Atom P+1. Die Anteile von Monosubstitutionsprodukten (Einfachverzweigung) am erfindungsgemäß hergestellten Olefingemisch liegen charakteristischerweise insgesamt im Bereich von 40 bis 75 Gew.-%, die Anteile an doppeltverzweigten Komponenten im Bereich von 5 bis 25 Gew.-%.

Es wurde ferner gefunden, daß die Dimerisienmgsgemische dann besonders gut weiter zu derivatisieren sind, wenn die Lage der Doppelbindung bestimmte Anforderungen erfüllt. In diesen vorteilhaften Olefingemischen ist die Lage der Doppelbindungen relativ zu den Verzweigungen dadurch charakterisiert, daß das Verhältnis der "aliphatischen" Wasserstoffatome zu "olefinischen" Wasserstoffatomen im Bereich H_{aliph.} : H_{olefin.} = (2*n-0,5) : 0,5 bis (2*n-1,9) : 1,9 liegt, wobei n die Kohlenstoffatom-Anzahl des bei der Dimerisierung erhaltenen Olefins ist.

(Als "aliphatische" Wasserstoffatome werden solche bezeichnet, die an Kohlenstoffatome gebunden sind, die an keiner C=C-Doppelbindung (π-Bindung) beteiligt sind, als "olefinische" Wasserstoffatome solche, die an ein Kohlenstoffatom gebunden ist, das eine π-Bindung betätigt.)

Besonders bevorzugt sind Dimerisierungsgemische, bei denen das Verhältnis
H_{aliph.} : H_{olefin.} = (2*n-1,0) : 1 bis (2*n-1,6) : 1,6 ist.

Die so hergestellten Olefingemische werden zunächst durch Umsetzung mit Kohlenmonoxid und Wasserstoff in Gegenwart von geeigneten, vorzugsweise kobalt- oder rhodiumhaltigen Katalysatoren zu Tensidalkoholen (Oxoalkoholen), verzweigten primären Alkoholen, hydroformyliert.

Eine gute Übersicht über das Verfahren der Hydroformylierung mit zahlreichen weiteren Literaturhinweisen findet sich beispielsweise in dem umfangreichen Aufsatz von Beller et al. in Journal of Molecular Catalysis, A104 (1995) 17-85 oder in Ulimanns Encyclopedia of Industrial Chemistry, Bd.A5 (1986), Seite 217 ff., Seite 333, sowie die diesbezüglichen Literaturverweise.

Die dort gegebenen umfassenden Informationen ermöglichen es dem Fachmann, auch die erfindungsgemäßen verzweigten Olefine zu hydroformylieren. Bei dieser Reaktion wird CO und Wasserstoff an olefinische Doppelbindungen angelagert, wobei gemäß dem folgenden Reaktionsschema Mischungen aus Aldehyden und Alkanolen erhalten werden:

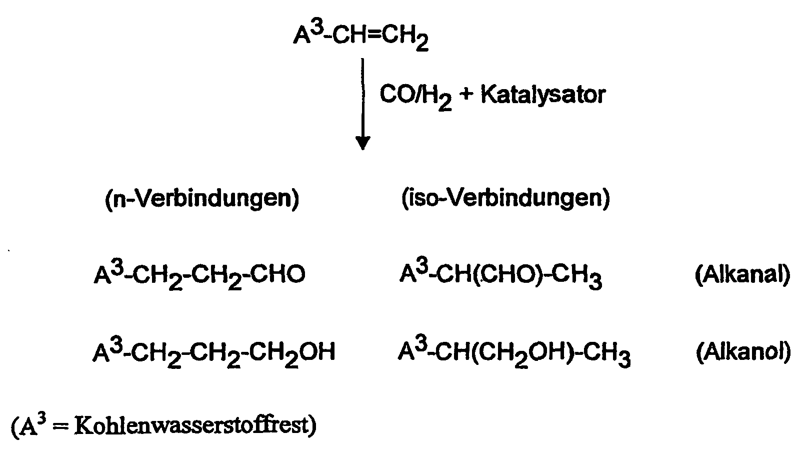

Das Molverhälnis von n- und iso-Verbindungen im Reaktionsgemisch liegt je nach den gewählten Verfahrensbedingungen der Hydroformylierung und dem eingesetzten Katalysator in der Regel im Bereich von 1:1 bis 20:1. Die Hydroformylierung wird normalerweise im Temperaturbereich von 90 bis 200°C und bei einem CO/H₂-Druck von 2,5 bis 35 MPa (25 bis 350 bar) ausgeführt. Das Mischungsverhältnis von Kohlenmonoxid zu Wasserstoff richtet sich danach, ob vorzugsweise Alkanale oder Alkanole erzeugt werden sollen. Man arbeitet zweckmäßigerweise im Bereich CO:H₂ von 10:1 bis 1:10, vorzugsweise 3:1 bis 1:3, wobei man zur Herstellung von Alkanalen den Bereich der niedrigen Wasserstoffparrialdrucke, zur Herstellung von Alkanolen den Bereich der hohen Wasserstoffpartialdrucke, z.B. CO:H₂ = 1:2, wählt.

Als Katalysatoren eignen sich vor allem Metallverbindungen der allgemeinen Formel HM(CO)₄ oder M₂(CO)₈, wobei M ein Metallatom, vorzugsweise ein Kobalt-, Rhodium- oder Rutheniumatom ist.

Im Allgemeinen werden unter Hydroformylierungsbedingungen aus den jeweils eingesetzten Katalysatoren oder Katalysatorvorstufen katalytisch aktive Spezies der allgemeinen Formel HₓM_{y}(CO)_{z}L_{q} gebildet, worin M für ein Metall der VIII. Nebengruppe, L für einen Liganden, der ein Phosphin, Phosphit, Amin, Pyridin oder jede andere Donorverbindung, auch in polymerer Form, sein kann, und q, x, y und z für ganze Zahlen, abhängig von der Wertigkeit und Art des Metalls sowie der Bindigkeit des Liganden L, stehen, wobei q auch 0 sein kann.

Bei dem Metall M handelt es sich vorzugsweise um Kobalt, Ruthenium, Rhodium, Palladium, Platin, Osmium oder Iridium und insbesondere um Kobalt, Rhodium oder Ruthenium.

Geeignete Rhodiumverbindungen oder Komplexe sind z.B. Rhodium(II)- und Rhodium(III)-salze, wie Rhodium(III)-chlorid, Rhodium(III)-nitrat, Rhodium(III)-sulfat, Kalium-Rhodium-sulfat, Rhodium(II) bzw. Rhodium(III)-carboxylat, Rhodium(II)- und Rhodium(III)-acetat, Rhodium(III)-oxid, Salze der Rhodium(III)-säure, wie z.B. Tnsammonium-hexaehlororhodat-(III). Weiterhin eignen sich Rhodiumkomplexe wie Rhodiumbiscarbonyl-acetylacetonat, Acetylacetonato-bisethylenRhodium-(I). Vorzugsweise werden Rhodiumbiscarbonyl-acetylacetonat oder Rhodiumacetat eingesetzt.

Geeignete Kobaltverbindungen sind zum Beispiel Kobalt(II)-chlorid, Kobalt(II)-sulfat, Kobalt(II)-carbonat, Kobalt(II)-nitrat, deren Amin- oder Hydratkomplexe, Kobaltcarboxylate wie Kobaltacetat, Kobaltethylhexanoat, Kobaltnaphthanoat, sowie der Kobaltcaprolactamat-Komplex. Auch hier können die Carbonylkomplexe des Kobalts wie Dikobaltoctocarbonyl, Tetrakobaltdodecacarbonyl und Hexakobalthexadecacarbonyl eingesetzt werden.

Die genannten Verbindungen des Kobalts, Rhodiums und Rutheniums sind im Prinzip bekannt und in der Literatur hinreichend beschrieben, oder sie können vom Fachmann analog zu den bereits bekannten Verbindungen hergestellt werden.

Die Hydroformylierung kann unter Zusatz von inerten Lösungs- oder Verdünnungsmitteln oder ohne solchen Zusatz durchgeführt werden. Geeignete inerte Zusätze sind beispielsweise Aceton, Methyl-ethylketon, Cyclohexanon, Toluol, Xylol, Chlorbenzol, Methylenchlorid, Hexan, Petrolether, Acetonitril sowie die hochsiedenden Anteile aus der Hydroformylierung der Dimerisierungsprodukte.

Sofern das erhaltene Hydroformylierungsprodukt einen zu hohen Aldchydgehalt aufweist, kann dieser auf einfache Weise durch eine Hydrierung, zum Beispiel mit Wasserstoff in Gegenwart von Raney-Nickel oder unter Verwendung anderer für Hydrierungsreaktionen bekannter, insbesondere Kupfer, Zink, Kobalt, Nickel, Molybdän, Zirkon oder Titan enthaltender Katalysatoren, beseitigt werden. Dabei werden die Aldehydanteile weitgehend zu Alkanolen hydriert. Eine praktisch restlose Beseitigung von Aldehydanteilen im Reaktionsgemisch läßt sich gewünschtenfalls durch Nachhydrierung, beispielsweise unter besonders schonenden und ökonomischen Bedingungen mit einem Alkaliborhydrid, erreichen.

Aus den so hergestellten Alkanolen können auf unterschiedliche Weise nichtionische oder anionische Tenside hergestellt werden.

Nichtionische Tenside werden erhalten durch Umsetzung der Alkanole mit Alkylenoxiden 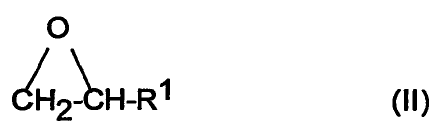 (Alkoxylierung) der Formel II
worin R¹ Wasserstoff oder ein geradkettiger oder verzweigter aliphatischer Rest der Formel CₙH₂ₙ₊₁ ist und n für eine Zahl von 1 bis 16, vorzugsweise von 1 bis 8 steht. Insbesondere bedeutet R¹ Wasserstoff, Methyl, Ethyl oder Propyl.

Die erfindungsgemäßen Alkanole können mit einer einzigen Alkylenoxid-Species oder mit mehreren verschiedenen umgesetzt werden. Bei der Umsetzung der Alkanole mit den Alkylenoxiden entstehen Verbindungen, die wiederum eine OH-Gruppe tragen und daher erneut mit einem Alkylenoxidmolekül reagieren können. Es werden daher je nach dem Molverhältnis von Alkanol zu Alkylenoxid Reaktionsprodukte erhalten, die mehr oder weniger lange Polyetherketten aufweisen. Die Polyetherketten können 1 bis ca. 200 Alkylenoxid-Baugruppen enthalten. Bevorzugt sind Verbindungen, deren Polyetherketten 1 bis 50 Alkylenoxid-Baugruppen enthalten.

Die Ketten können aus gleichen Kettengliedern bestehen, oder sie können verschiedene Alkylenoxid-Baugruppen aufweisen, die sich bezüglich ihres Restes R¹ voneinander unterscheiden. Diese unterschiedlichen Baugruppen können innnerhalb der Kette in statistischer Verteilung oder in Form von Blöcken vorliegen.

Das folgende Reaktionsschema soll die Alkoxylierung der oben beschriebenen Alkanole am Beispiel einer Umsetzung mit zwei verschiedenen Alkylenoxiden, die in unterschiedlichen Mol-Mengen x und y eingesetzt werden, veranschaulichen.

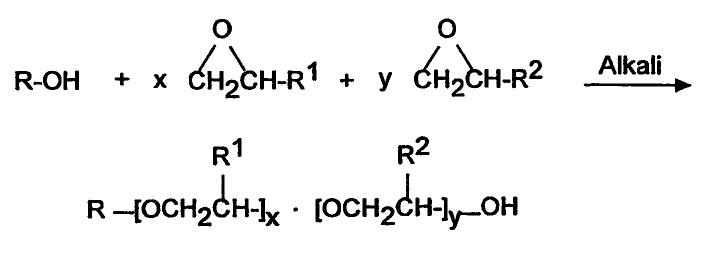

R¹ und R² sind verschiedene Reste im Rahmen der oben für R¹ gegebenen Definitionen und R-OH ist ein oben beschriebener verzweigter Oxoalkohol.

Vorzugsweise wird die Alkoxylierung durch starke Basen katalysiert, die zweckmäßigerweise in Form eines Alkalihydroxids oder Erdalkalihydroxids, in der Regel in einer Menge von 0,1 bis 1 Gew.-% bezogen auf die Menge des Alkanols R²-OH, zugesetzt werden. (Vergl. G.Gee et al., J. Chem. Soc. (1961), S. 1345; B. Wojtech, Makromol. Chem. **66**, (1966), S.180)

Auch eine saure Katalyse der Additionsreaktion ist möglich. Neben Bronstedsäuren eignen sich auch Lewissäuren wie z.B. AlCl₃ oder BF₃. (Vergl. P.H.Plesch, The Chemistry of Cationic Polymerization, Pergamon Press, New York (1963)).

Die Additionsreaktion wird bei Temperaturen von etwa 120 bis etwa 220°C, vorzugsweise von 140 bis 160°C, im geschlossenen Gefäß ausgeführt. Das Alkylenoxid oder die Mischung verschiedener Alkylenoxide wird der Mischung aus erfindungsgemäßem Alkanolgemisch und Alkali unter dem bei der gewählten Reaktionstemperatur herrschenden Dampfdruck des Alkylenoxidgemisches zugeführt. Gewünschtenfalls kann das Alkylenoxid mit bis zu etwa 30 bis 60 % mit einem Inertgas verdünnt werden. Dadurch wird eine zusätzliche Sicherheit gegen explosionsartige Polyaddition des Alkylenoxids gegeben.

Wird ein Alkylenoxidgemisch eingesetzt, so werden Polyetherketten gebildet in denen die verschiedenen Alkylenoxidbausteine praktisch statistisch verteilt sind. Variationen in der Verteilung der Bausteine längs der Polyetherkette ergeben sich aufgrund unterschiedlicher Reaktionsgeschwindigkeiten der Komponenten und können auch willkürlich durch kontinuierliche Zufuhr einer Alkylenoxidmischung programmgesteuerter Zusammensetzung erreicht werden. Werden die verschiedenen Alkylenoxide nacheinander zur Reaktion gebracht so erhält man Polyetherketten, mit blockartiger Verteilung der Alkylenoxid-Bausteine.

Die Länge der Polyetherketten schwankt innerhalb des Reaktionsprodukts statistisch um einen Mittelwert, der im wesentlichen dem sich aus der Zusatzmenge ergebenden stöchiometrischen Wert entspricht.

Sowohl die aus dem Syntheseschritt d) hervorgehenden Alkanolgemische als auch die daraus im Syntheseschritt e) gegebenenfalls hergestellten Polyether können in anionische Tenside überführt werden indem man sie in an sich bekannter Weise mit Schwefelsäure oder Schwefelsäurederivaten zu sauren Alkylsulfaten oder Alkylethersulfaten oder mit Phosphorsäure oder ihren Derivaten zu sauren Alkylphosphaten bzw. Alkyletherphosphaten verestert (sulfatiert bzw. phosphatiert).

Sulfatierungsreaktionen von Alkoholen sind bereits beschrieben worden z.B. in US-A-3 462 525, 3 420 875 oder 3 524 864. Details zur Durchführung dieser Reaktion finden sich auch in "Ullmanns Encyclopedia of Industrial Chemistry", 5.Auflage Bd. A25 (1994), Seiten 779-783 und in den dort angegebenen Literaturverweisen.

Wird Schwefelsäure selbst zur Veresterung eingesetzt, so verwendet man zweckmäßigerweise eine 75 bis 100 gew.-%ige, vorzugsweise 85 bis 98 gew.-%ige Säure (sog. "konzentrierte Schwefelsäure" oder "Monohydrat"). Die Veresterung kann in einem Lösungs- oder Verdünnungsmittel vorgenommen werden, wenn es für die Steuerung der Reaktion, z.B. der Wärmeentwicklung erwünscht ist. In der Regel wird der alkoholische Reaktant vorgelegt, und das Sulfatierungsmittel unter ständigem Durchmischen allmählich zugefügt. Wenn eine vollständige Veresterung der Alkoholkomponente gewünscht wird verwendet man das Sulfatierungsmittel und den Alkanol im Molverhältnis von 1:1 bis 1:1,5, vorzugsweise von 1:1 bis 1:1,2. Geringere Mengen von Sulfatierungsmittel können vorteilhaft sein, wenn Mischungen von erfindungsgemäßen Alkanol-alkoxylaten eingesetzt und Kombinationen von Neutralen und anionischen Tensiden hergestellt werden sollen. Die Veresterung wird normalerweise bei Temperaturen von Zimmertemperatur bis 85°C, vorzugsweise im Bereich von 45 bis 75°C, ausgeführt.

Gegebenenfalls kann es zweckmäßig sein, die Veresterung in einem niedrig siedenden, mit Wasser nicht mischbaren Lösungs- und Verdünnungsmittel bei dessen Siedepunkt auszuführen, wobei das bei der Veresterung entstehende Wasser azeotrop abdestilliert wird.

Anstelle von Schwefelsäure der oben angegebenen Konzentration kann zur Sulfatierung der erfindungsgemäßen Alkanolgemische auch beispielsweise Schwefeltrioxid, Schwefeltrioxid-Komplexen, Lösungen von Schwefeltrioxid in Schwefelsäure ("Oleum"), Chlorsulfonsäure, Sulfurylchlorid oder auch Amidosulfonsäure eingesetzt werden. Die Reaktionsbedingungen sind dann zweckentsprechend anzupassen.

Wird Schwefeltrioxid als Sulfatierungsmittel eingesetzt so kann die Reaktion auch vorteilhafterweise in einem Fallfilmreaktor im Gegenstrom, gewünschtenfalls auch kontinuierlich, ausgeführt werden.

Die Ansätze werden nach der Veresterung durch Alkalizusatz neutralisiert und, ggf. nach Entfernung überschüssigen Alkalisulfates und eventuell vorhandener Lösungsmittel, aufgearbeitet.

In analoger Weise können Alkanole und Alkanolether und deren Gemische mit Phosphatierungsagenzien auch zu sauren Phosphorsäureestern umgesetzt (phösphatiert) werden.

Als Phosphatierungsagenzien eignen sich vornehmlich Phosphorsäure, Polyphosphorsäure und Phosphorpentoxid, aber auch POCl₃, wenn anschließend eine Hydrolyse der verbleibenden Säurechloridfunktionen vorgenommen wird. Die Phosphatierung von Alkoholen ist beispielsweise in Synthesis 1985, Seiten 449 bis 488 beschrieben worden.

Die folgenden Ausführungsbeispiele veranschaulichen die Herstellung und Verwendung der erfindungsgemäß einzusetzenden Tenside.

### Beispiel 1, Herstellung von C₅/C₆-Olefinen aus C₄-Olefin-Strömen durch Metathese

Eine butadienfreie C₄-Fraktion mit einem Gesamthutengehalt von 84,2 Gew.-% und einem Molverhältnis 1-Buten : 2-Buten von 1,06 ("Raffinat II") wird bei 40°C und 10 bar kontinuierlich durch einen mit Re₂O₇/Al₂O₃-Heterogenkontakt beschickten Rohrreaktor geleitet. Die Katalysatorbelastung wird auf 4500 kg/(m2*h) eingestellt. Der Reaktoraustrag wird destillativ getrennt und enthält folgende Komponenten (Angaben in Massenprozent):
Ethen: 1,15 %, Propen: 18,9 %, Butan: 15,8 %, 2-Buten: 19,7 %, 1-Buten: 13,3 %, i-Buten: 1,00 %, 2-Penten: 19,4 %, Methylbuten: 0,45 %, 3-Hexen 10,3 %.

### Beispiele 2A und 2B: Heterogenkatalysierte Dimerisierung von 3-Hexen.

### 2A. Festbettverfahren.

Ein isotherm beheizbarer Reaktor mit 16 mm Durchmesser wurde mit 100 ml eines Katalysators der folgenden Zusammensetzung befüllt:
50 Gew.-% NiO, 34 Gew.-% SiO₂, 13 Gew.-% TiO₂, 3 Gew.-% Al₂O₃ (gemäß DE-A-43 39 713), 24 Stdn. bei 160°C in N₂ konditioniert, eingesetzt als 1 bis 1,5 mm Splitt.

Es wurden 5 Versuche gemacht, wobei durch das festgelegte Katalysatorbett 3-Hexen (99,9 gew.-%ig, 0,1 Gew.-% C₇ bis C₁₁-Fraktionen) in einer auf das Reaktorvolumen bezogenen Rate (WHSV) von 0,25 kg/l*h geleitet und in einer Rate von 24 bis 28 g/h ausgeschleust wurde. Variiert wurde bei den einzelnen Versuchen die Reaktionstemperatur oder die Betriebsdauer des Versuchs.

Die folgende Tabelle 1 zeigt die Versuchsbedingungen der fünf Versuche und die dabei erhaltenen Ergebnisse.

**Tabelle 1.**

| Verfahrensbedingungen und Ergebnisse beim Festbettverfahren | | | | | | |
|---|---|---|---|---|---|---|
| Reaktionsbedingungen | | | | | | |
| Temperatur [°] | 100 | 120 | 140 | 160 | 160 | C₁₂-Destillat |
| Druck [bar] | 20 | 20 | 20 | 25 | 25 | |
| Betriebsstunden | 12 | 19 | 36 | 60 | 107 | |
| Flüssiganfall [g/h] | 24 | 27 | 27 | 28 | 27 | |

| Zusammensetzung Gew.-% | | | | | | |
|---|---|---|---|---|---|---|
| C₆ | 68,5 | 52,7 | 43,6 | 57,0 | 73,2 | 0,1 |
| C₇ - C₁₁ | 0,2 | 0,2 | 0,3 | 0,2 | 0,2 | - |
| C₁₂ | 25,9 | 38,6 | 44,0 | 35,6 | 23,6 | 99,9 |
| > C₁₂ | 5,4 | 8,5 | 12,1 | 7,2 | 3,0 | - |
| Umsatz | 31,4 | 47,2 | 56,4 | 42,9 | 26,7 | - |
| C₁₂-Selektivität [Gew,-%] | 82,5 | 81,8 | 78,2 | 83,0 | 88,4 | - |
| S-Gehalt im Flüssiganfall [ppm] | - | - | - | - | - | - |

Das ausgeschleuste Produkt wurde fraktioniert destilliert und eine Bestimmung der Gerüstisomeren der C₁₂-Fraktion durchgeführt. Die Analyse ergab 14,2 Gew.-% n-Dodecene, 31,8 Gew.-% 5-Methylundecene, 29,1 Gew.-% 4-Ethyldecene, 6,6 Gew.-% 5,6-Dimethyldecene, 9,3 Gew.-% 4-Methyl-5-ethylnonene und 3,7 Gew.-% Diethyloctene.

### B. Suspensionsverfahren (Fließbeitverfahren)

Ein isotherm beheizbaren Reaktor mit 20 mm Durchmesser und einem Volumen von 157 ml wurde mit 30 g eines Katalysators der folgenden Zusammensetzung befüllt:
50 Gew.-% NiO, 34 Gew.-% SiO₂, 13 Gew.-% TiO₂, 3 Gew.-% Al₂O₃ (gemäß DE-A-43 39 713), 24 Stdn. bei 160°C in N₂ konditioniert, eingesetzt als 0,05 bis 0,5 mm Sprühgut.

Es wurden 6 Versuche gemacht wobei durch das Katalysator-Fließbett von unten her 3-Hexen (99,9 gew.-%ig, 0,1 Gew.-% C₇ bis C₁₁-Fraktionen) in einer auf das Reaktorvolumen bezogenen Rate von 0,25 kg/l*h geleitet wurde. Das aus dem Reaktor austretende Reaktionsprodukt wurde großteils rückgeführt (Rückführung:Feedmenge zwischen ca.45 und 60 variiert). Variiert wurde bei den einzelnen Versuchen auch die Reaktionstemperatur, die Feedmenge, die Kreislaufströmung, die Rückführrate und die WHSV des Versuchs. Die Versuchsdauer betrug 8 Stunden.

Die folgenden Tabellen 2A und 2B zeigen die Versuchsbedingungen der sechs Versuche und die dabei erhaltenen Ergebnisse.

### Tabellen 2

Versuchsbedingungen und Ergebnisse beim Suspensionsverfahren.

**Tabelle 2A:**

| Versuchsbedingungen | | | | | | | |
|---|---|---|---|---|---|---|---|
| Versuch Nr. | Temperatur [°C] | Druck [bar] | Feed [g/h] | Kreislauf [g/h] | Rückführrate [X:1] | WHSV | Betriebsdauer [h] |
| 1 | 130 | 11,0 | 20 | 1200 | 60,0 | 0,127 | 8 |
| 2 | 130 | 11,0 | 23 | 1200 | 52,2 | 0,146 | 8 |
| 3 | 130 | 12,0 | 21 | 1100 | 52,4 | 0,134 | 8 |
| 4 | 130 | 12,2 | 24 | 1100 | 45,8 | 0,153 | 8 |
| 5 | 140 | 13,4 | 23 | 1180 | 51,3 | 0,146 | 8 |
| 6 | 150 | 14,1 | 22 | 1200 | 54,5 | 0,140 | 8 |

**Tabelle 2B:**

| Zusammensetzung des Reaktionsaustrags | | | | | | | |
|---|---|---|---|---|---|---|---|
| Versuch Nr. | % C₆ | % >C₆ | % C₁₂ | % C₁₈ | % C₂₄ | % Umsatz | % C₁₂-Selektivität |
| 1 | 83,9 | 0,5 | 14,3 | 1,1 | 0,2 | 16,1 | 88,82 |
| 2 | 80,5 | 0,5 | 16,9 | 1,8 | 0,3 | 19,5 | 86,67 |
| 3 | 80,3 | 0,4 | 17,0 | 1,9 | 0,3 | 19,7 | 86,29 |
| 4 | 81,6 | 0,5 | 15,5 | 2,0 | 0,3 | 18,4 | 84,24 |
| 5 | 75,9 | 0,5 | 20,4 | 2,6 | 0,5 | 24,1 | 84,65 |
| 6 | 71,1 | 0,6 | 24,0 | 3,5 | 0,7 | 28,9 | 83,04 |

Das ausgeschleuste Produkt wurde fraktioniert destilliert und eine Bestimmung der Gerüstisomeren der C₁₂-Fraktion durchgeführt. Die Analyse ergab 14 Gew.-% n-Dodecene, 32 Gew.-% 5-Methylundecene, 29 Gew.-% 4-Ethyldecene, 7 Gew.-% 5,6-Dimethyldecene, 9 Gew.-% 4-Methyl-5-ethylnonene und 4 Gew.-% Diethyloctene.

### Beispiel 3, Hydroformylierung einer Dodecenmischung

750 g des gemäß Beispiel 2B hergestellten Dodecengemisches werden mit 3,0 g Co₂(CO)₈ bei 185°C und 280 bar CO/H₂ (Vol.-Verhältnis = 1:1,5) unter Zusatz von 75 g H₂O in einem 2,5 1 Hubrührautoklaven 5 Stunden hydroformyliert. Der Reakdonsaustrag wird mit 10 gew.-%iger Essigsäure unter Lufteinleitung bei 90°C oxidativ entkobaltet. Das Oxoprodukt wird unter Zusatz von 10 Gew.-% Wasser in einem 2,5 1 Hubrührautoklaven mit 50 g Raney-Nickel bei 125°C und 280 bar Wasserstoffdruck 10 Stunden hydriert. Der Reaktionsaustrag wird fraktioniert destilliert.

450 g einer auf diese Weise hergestellten Tridecanolfiaktion werden mit 3,5 g NaBH₄ nachhydriert.

Die OH-Zahl des erhaltenen Tridecanols beträgt 277 mg KOH/g.

¹H-NMR-spektroskopisch wurde ein mittlerer Verzweigungsgrad von 2,3 Methylgruppen/Molekül bestimmt, entsprechend einem Verzweigungsgrad von 1,3.

### Beispiel 3A, Hydroformylierung einer Dodecenmischung

2,12 kg des gemäß Beispiel 2A hergestellten Dodecengemisches werden mit 8 g Co₂(CO)₈ bei 185°C und 280 bar CO/H₂ (Volumenverhältnis 1:1) unter Zusatz von 210 g Wasser in einem 5-1 Drehruhrautoklaven 5 Stunden hydroformyliert. Der Reaktionsaustrag wird mit 10 gew.-%iger Essigsäure unter Lufteinleitung bei 90°C oxidativ entkobaltet. Das erhaltene Oxoprodukt wird in einem 5-1 Rohrreaktor in Rieselfahrweise an einem Co/Mo-Festbettkatalysator bei 175°C und 280 bar Wasserstoffdruck unter Zusatz von 10 Gew.-% Wasser hydriert. Das Alkoholgemisch wird destillativ aufgearbeitet. Das erhaltene Tridecanol hat eine OH-Zahl von 279 mg KOH/g; ¹H-NMR-spektroskopisch wird ein mittlerer Verzweigungsgrad von 1,53 gemessen.

### Beispiel 3B, Hydroformylierung einer Dodecenmischung

50 mg Rhodiumbiscarbonyl-acetylacetonat, 4,5 g eines Polyethylenimins der Molmasse M_{w} = 460000, bei dem 60 % aller Stickstoffatome mit Laurinsäure acyliert sind, 800 g eines gemäß Beispiel 2A hergestellten Dodecengemisches und 196 g Toluol werden in einem 2,5-1 Hubrührautoklaven unter einem Druck von 280 bar CO/H₂ (Volumenverhältnis 1:1) 7 Stunden auf 150°C erhitzt. Dann wird der Autoklav abgekühlt, entspannt und entleert.Durch Gaschromatographie des erhaltenen Reaktionsprodukts wird ein Umsatz des Olefins von 93 % festgestellt. Das erhaltene Oxoprodukt wird in einem 2,5-1 Rohrreaktor in Rieselfahrweise an einem Co/Mo-Festbettkatalysator bei 175°C und 280 bar Wasserstoffdruck unter Zusatz von 10 Gew.-% Wasser hydriert und das entstandene Alkoholgemisch destillativ aufgearbeitet. Das erhaltene Tridecanol hat eine OH-Zahl von 279 mg KOH/g; ¹H-NMR-spektroskopisch wird ein mittlerer Verzweigungsgrad von 1,63 gemessen.

### Beispiel 3C, Hydroformylierung einer Dodecenmischung

50 mg Rhodiumbiscarbonyl-acetylacetonat, 4,5 g eines Polyethylenimins der Molmasse M_{w} = 460000, bei dem 60 % aller Stickstoffatome mit Laurinsäure acyliert sind, 800 g eines gemäß Beispiel 2A hergestellten Dodecengemisches und 196 g Toluol werden in einem 2,5-1 Hubrührautoklaven unter einem Druck von 280 bar CO/H₂ (Volumenverhältnis 1:1) 7 Stunden auf 160°C erhitzt. Dann wird der Autoklav abgekühlt, entspannt und entleert.Durch Gaschromatographie des erhaltenen Reaktionsprodukts wird ein Umsatz des Olefins von 94 % festgestellt. Das erhaltene Oxoprodukt wird in einem 2,5-1 Rohrreaktor in Rieselfahrweise an einem Co/Mo-Festbettkatalysator bei 175°C und 280 bar Wasserstoffdruck unter Zusatz von 10 Gew.-% Wasser hydriert und das entstandene Alkoholgemisch destillativ aufgearbeitet. Das erhaltene Tridecanol hat eine OH-Zahl von 279 mg KOH/g; ¹H-NMR-spektroskopisch wird ein mittlerer Verzweigungsgrad von 1,69 gemessen.

### Beispiel 4, Herstellung eines C₁₃-Oxoalkoholethoxilates mit 2,75 mol Ethylenoxid

500 g C₁₃-Oxoalkohol (hergestellt gemäß Beispiel 3B) werden mit 1,2 g NaOH in einen trockenen 21-Autoklaven eingefüllt. Der Autoklaveninhalt wird auf 150°C erhitzt und 300 g Ethylenoxid unter Druck in den

Autoklaven gepreßt. Nachdem sich die gesamte Ethylenoxidmenge im Autoklaven befindet, wird der Autoklav für 30 Minuten bei 150°C gehalten. Nach dem Abkühlen wird der Katalysator mit Essigsäure neutralisiert.

Das erhaltene Tensid weist einen Trübungspunkt von 35°C, gemessen 1 %ig in 10 %iger Butyldiglykollösung nach DIN 53917, auf. Die Oberflächenspannung bei einer Konzentration von 1g/l beträgt 25,8 mN/m, gemessen nach DIN 53914.

### Beispiel 5, Herstellung eines C₁₃-Oxoalkoholethoxilates mit 5 mol Ethylenoxid

400 g C₁₃-Oxoalkohol (hergestellt gemäß Beispiel 3B) werden mit 1,5 g NaOH in einen trockenen 2 1-Autoklaven eingefülit. Der Autoklaveninhalt wird auf 150°C erhitzt und 440 g Ethylenoxid unter Druck in den Autoklaven gepreßt. Nachdem sich die gesamte Ethylenoxidmenge im Autoklaven befindet, wird der Autoklav für 30 Minuten bei 150°C gehalten. Nach dem Abkühlen wird der Katalysator mit Essigsäure neutralisiert.

Das erhaltene Tensid weist einen Trübungspunkt von 58,5°C, gemessen 1 %ig in 10 %iger Butyldiglykollösung nach DIN 53917, auf. Die Oberflächenspannung bei einer Konzentration von 1g/l beträgt 26,7 mN/m, gemessen nach DIN 53914.

### Beispiel 6, Herstellung eines C₁₃-Oxoalkoholethoxilates mit 7,5 mol Ethylenoxid

600 g C₁₃-Oxoalkohol (hergestellt gemäß Beispiel 3B) werden mit 1,6 g NaOH in einen trockenen 2 1-Autoklaven eingefüllt. Der Autoklaveninhalt wird auf 140°C erhitzt und 990 g Ethylenoxid unter Druck in den Autoklaven gepreßt. Nachdem sich die gesamte Ethylenoxidmenge im Autoklaven befindet, wird der Autoklav für 40 Minuten bei 140°C gehalten. Nach dem Abkühlen wird der Katalysator mit Essigsäure neutralisiert.

Das erhaltene Tensid weist einen Trübungspunkt von 74°C, gemessen 1 %ig in 10 %iger Butyldiglykollösung nach DIN 53917, auf. Die Oberflächenspannung bei einer Konzentration von 1g/l beträgt 26,6 mN/m, gemessen nach DIN 53914.

### Beispiel 7, Herstellung eines C₁₃-Oxoalkoholethoxilates mit 9,5 mol Ethylenoxid

300 g C₁₃-Oxoalkohol (hergestellt gemäß Beispiel 3B) werden mit 1,8 g NaOH in einen trockenen 2 1-Autoklaven eingefüllt. Der Autoklaveninhalt wird auf 150°C erhitzt und 630 g Ethylenoxid unter Druck in den

Autoklaven gepreßt. Nachdem sich die gesamte Ethylenoxidmenge im Autoklaven befindet, wird der Autoklav für 50 Minuten bei 150°C gehalten. Nach dem Abkühlen wird der Katalysator mit Essigsäure neutralisiert.

Das erhaltene Tensid weist einen Trübungspunkt von 66°C, gemessen 1 %ig in Wasser nach DIN 53917, auf. Die Oberflächenspannung bei einer Konzentration von 1g/l beträgt 27,9 mN/m, gemessen nach DIN 53914.

### Beispiel 8, Herstellung eines C₁₃-Oxoalkoholethoxilates mit 2 mol Ethylenoxid

500 g C₁₃-Oxoalkohol (hergestellt gemäß Beispiel 3B) werden mit 1,0 g NaOH in einen trockenen 2 1-Autoklaven eingefüllt. Der Autoklaveninhalt wird auf 120°C erhitzt und 220 g Ethylenoxid unter Druck in den Autoklaven gepreßt. Nachdem sich die gesamte Ethylenoxidmenge im Autoklaven befindet, wird der Autoklav für 30 Minuten bei 120°C gehalten. Nach dem Abkühlen wird der Katalysator mit Schwefelsäure neutralisiert.

Das erhaltene Tensid weist einen Trübungspunkt von 21 °C, gemessen 1% ig in 10 %iger Butyldiglykollösung nach DIN 53917, auf. Die Oberflächenspannung bei einer Konzentration von 1g/l beträgt 26,6 mN/m, gemessen nach DIN 53914.

### Beispiel 9, (Vergleich) Herstellung eines C₁₃-Oxoalkoholethoxilates mit 2,75 mol Ethylenoxid

500 g C₁₃-Oxoalkohol (hergestellt durch Trimerisierung von n-Butenen und anschließender Hydroformylierung) werden mit 1,2 g NaOH in einen trockenen 21-Autoklaven eingefüllt. Der Autoklaveninhalt wird auf 150°C erhitzt und 300 g Ethylenoxid unter Druck in den Autoklaven gepreßt. Nachdem sich die gesamte Ethylenoxidmenge im Autoklaven befindet, wird der Autoklav für 30 Minuten bei 150°C gehalten. Nach dem Abkühlen wird der Katalysator mit Essigsäure neutralisiert.

Das erhaltene Tensid weist einen Trübungspunkt von 39,5°C, gemessen 1% ig in 10 %iger Butyldiglykollösung nach DIN 53917, auf. Die Oberflächenspannung bei einer Konzentration von 1g/l beträgt 26,7 mN/m, gemessen nach DIN 53914.

### Beispiel 10, (Vergleich) Herstellung eines C₁₃-Oxoalkoholethoxilates mit 5 mol Ethylenoxid

400 g C₁₃-Oxoalkohol (hergestellt durch Trimerisierung von n-Butenen und anschließender Hydroformylierung) werden mit 1,5 g NaOH in einen trockenen 21-Autoklaven eingefüllt. Der Autoklaveninhalt wird auf 150°C erhitzt und 440 g Ethylenoxid unter Druck in den Autoklaven gepreßt. Nachdem sich die gesamte Ethylenoxidmenge im Autoklaven befindet, wird der Autoklav für 30 Minuten bei 150°C gehalten. Nach dem Abkühlen wird der Katalysator mit Essigsäure neutralisiert.

Das erhaltene Tensid weist einen Trübungspunkt von 61°C, gemessen 1 %ig in 10 %iger Butyldiglykollösung nach DIN 53917, auf. Die Oberflächenspannung bei einer Konzentration von 1g/l beträgt 27,2 mN/m, gemessen nach DIN 53914.

### Beispiel 11, (Vergleich) Herstellung eines C₁₃-Oxoalkoholethoxilates mit 7 mol Ethylenoxid

400 g C₁₃-Oxoalkohol (hergestellt durch Trimerisierung von n-Butenen und anschließender Hydroformylierung) werden mit 1,6 g NaOH in einen trockenen 2 1-Autoklaven eingefüllt. Der Autoklaveninhalt wird auf 150°C erhitzt und 440 g Ethylenoxid unter Druck in den Autoklaven gepreßt. Nachdem sich die gesamte Ethylenoxidmenge im Autoklaven befindet, wird der Autoklav für 30 Minuten bei 150°C gehalten. Nach dem Abkühlen wird der Katalysator mit Essigsäure neutralisiert.

Das erhaltene Tensid weist einen Trübungspunkt von 70°C, gemessen 1 %ig in 10 %iger Butyldiglykollösung nach DIN 53917, auf Die Oberflächenspannung bei einer Konzentration von 1g/l beträgt 27,5 mN/m, gemessen nach DIN 53914.

### Beispiel 12, (Vergleich) Herstellung eines C₁₃-Oxoalkoholethoxilates mit 9 mol Ethylenoxid

400 g C₁₃-Oxoalkohol (hergestellt durch Trimerisierung von n-Butenen und anschließender Hydroformylierung) werden mit 1,7 g NaOH in einen trockenen 2 1-Autoklaven eingefüllt. Der Autoklaveninhait wird auf 150°C erhitzt und 440 g Ethylenoxid unter Druck in den Autoklaven gepreßt. Nachdem sich die gesamte Ethylenoxidmenge im Autoklaven befindet, wird der Autoklav für 30 Minuten bei 150°C gehalten. Nach dem Abkühlen wird der Katalysator mit Essigsäure neutralisiert.

Das erhaltene Tensid weist einen Trübungspunkt von 60°C, gemessen 1 %ig in Wasser nach DIN 53917, auf. Die Oberflächenspannung bei einer Konzentration von 1g/l beträgt 28,2 mN/m, gemessen nach DIN 53914.

### Beispiel 13, (Vergleich) Herstellung eines C₁₃-Oxoalkoholethoxilates mit 9,7 mol Ethylenoxid

400 g C₁₃-Oxoalkohol (hergestellt durch Trimerisierung von n-Butenen und anschließender Hydroformylierung) werden mit 1,8 g NaOH in einen trockenen 2 1-Autoklaven eingefüllt. Der Autoklaveninhait wird auf 150°C erhitzt und 440 g Ethylenoxid unter Druck in den Autoklaven gepreßt. Nachdem sich die gesamte Ethylenoxidmenge im Autoklaven befindet, wird der Autoklav für 30 Minuten bei 150°C gehalten. Nach dem Abkühlen wird der Katalysator mit Essigsäure neutralisiert.

Das erhaltene Tensid weist einen Trübungspunkt von 70°C, gemessen 1 %ig Wasser nach DIN 53917, auf. Die Oberflächenspannung bei einer Konzentration von 1g/l beträgt 28,8 mN/m, gemessen nach DIN 53914.

### Beispiel 14, Waschtest

Die Waschversuche wurden in einem Launderometer LP 2 der Fa. Atlas mit folgender Rezeptur durchgeführt:

| | |
|---|---|
| 19 Gew-% | Tensid |
| 30 Gew-% | Zeolith A |
| 12 Gew-% | Natriumcarbonat |
| 3 Gew-% | Natriumsilikat |
| 1,2 Gew-% | Carboxymethylcellulose (Tylose® CR 1500 p (bezogen auf Wirksubstanz)) |
| 14,4 Gew-% | Natriumperborat-monohydrat |
| 4 Gew-% | TAED |
| 5 Gew-% | Polycarboxylat (Sokalan® CP 5) |
| 0,5 Gew-% | Seife |
| 4 Gew-% | Natriumsulfat |
| 6,9 Gew-% | Wasser |

| | |
|---|---|
| Waschmittelmenge | 4 g/l |
| Flottenverhältnis | 1 : 12,5 |
| Wasserhärte | 3 mmol |
| Ca : Mg | 4:18 |
| Waschdauer | 30 min |

Die Waschversuche wurden mit Testmaterial EMPA 101 (Hersteller: EMPA Testmaterials, Mövenstraße 12, CH 9015 St.Gallen) und mit Testgewebe wfk 10 D (Hersteller:wfk-Testgewebe GmbH, Christenfeld 10, 41375 Brueggen) durchgeführt.

### Anschmutzung:

| | |
|---|---|
| EMPA 101 | Ruß/Olivenöl auf Baumwolle |
| wfk 10 D | wfk-Pigment/Hautfett auf Baumwolle. |

Die folgende Tabelle zeigt die bei den Waschversuchen bei 40°C und bei 60°C erhaltenen Ergebnisse. Angegeben sind jeweils die nach der Wäsche gemessenen Remissionsgrade in [%] der eingestrahlten Lichtmenge.

Der Remissionsgrad wird gemessen mit dem Gerät Elrepho 2000 der Fa. datacolor AG.

**Tabelle,**

| Waschergebnisse. | | | |
|---|---|---|---|
| | Waschtemperatur [°C] | Remissionsgrad [%] bei EMPA 101 | Remissionsgrad [%] bei wfk 10 D |
| Beispiel 4 | 40 | 31 | 59 |
| Beispiel 5 | 40 | 28 | 51 |
| Beispiel 6, (Vergleich) | 40 | 28 | 51 |
| Beispiel 7, (Vergleich) | 40 | 26 | 51 |
| Beispiel 4 | 60 | 37 | 63 |
| Beispiel 5 | 60 | 35 | 53 |
| Beispiel 6, (Vergleich) | 60 | 34 | 57 |
| Beispiel 7, (Vergleich) | 60 | 31 | 51 |

Die Figuren 2a und 2b veranschaulichen den in der Tabelle des Beispiels 8 angegebenen Zusammenhang zwischen dem auf der Ordinate aufgetragenen Alkoxylierungsgrad (Alkylenoxid-Gehalt) in mol Alkylenoxid pro mol alkoxylierte Verbindung (EO-[mol/mol]) und dem auf der Abszisse aufgetragenen Waschergebnis - ausgedrückt durch den Remissionsgrad in %.

Die Figur 2a zeigt das Waschergebnis bei Einsatz des EMPA 101 - Testmaterials, die Figur 2b zeigt das Waschergebnis bei Einsatz des wfk 10 D - Testmaterials.

Die in den Figuren 2a und 2b angegebenen Bezugszeichen haben folgende Bedeutungen:
E40 Wäsche mit erfindungsgemäßen Tensiden bei 40°C,
E60 Wäsche mit erfindungsgemäßen Tensiden bei 60°C,
V40 Wäsche mit nächstvergleichbaren bekannten Tensiden bei 40°C,
V60 Wäsche mit nächstvergleichbaren bekannten Tensiden bei 60°C.

### Beispiel 15, Bestimmung der Ölablösung

Kupferplättchen (2 x 6 cm) werden möglichst genau mit 0,1 g Mineralöl SN 200 beaufschlagt. Diese Plättchen werden vorsichtig in eine 0,1%ige Tensidlösung aus den Beispielen 4 - 7 und den Vergleichen 9 - 13 eingetaucht. Die Tensidlösung wird nicht gerührt. Nach 25 Minuten werden die Plättchen wieder aus der Tensidlösung entfernt, im Trockenschrank bei 70°C 5 Stunden getrocknet und die Ölmenge zurückgewogen. Die Ergebnisse sind in Tabelle 1 zusammengefasst und in der Figur 3 graphisch dargestellt

**Tabelle:**

| **Ölablösung auf Kupfer** | |
|---|---|
| Tensid, gemäß | Abgelöste Ölmenge, %, bezogen auf Anfangsmenge. |
| Beispiel 4 | 81 |
| Beispiel 5 | 80,8 |
| Beispiel 6 | 72,9 |
| Beispiel 7 | 79,4 |
| Vergleich 9 | 54,2 |
| Vergleich 10 | 73,8 |
| Vergleich 11 | 62,8 |
| Vergleich 12 | 60,1 |
| Vergleich 13 | 57,5 |

Die Figur 3 veranschaulicht den in der obigen Tabelle angegebenen Zusammenhang zwischen dem auf der Ordinate aufgetragenen Alkoxylierungsgrad (Alkylenoxid-Gehalt) in mol Alkylenoxid pro mol alkoxylierte Verbindung und der auf der Abszisse aufgetragenen Reinigungswirkung - ausgedrückt durch die Ölablösung in % der aufgetragenen Ölmenge.

### Beispiel 16, Herstellung eines Alkylphosphates

300 g C₁₃-Oxoalkohol (hergestellt durch Dimerisierung von 3-Hexen und anschließender Hydroformylierung) werden in einem Rührgefäß unter Stickstoff auf 60°C erwärmt und langsam mit 125 g Polyphosphorsäure versetzt. Dabei sollte die Temperatur 65°C nicht übersteigen. Gegen Ende der Zugabe wird die Temperatur auf 70°C erhöht und eine Stunde bei dieser Temperatur gerührt.

Die Oberflächenspannung bei einer Konzentration von 1g/l beträgt 35,1 mN/m, gemessen nach DIN 53914.

### Beispiel 17, Herstellung eines Alkyletherphosphates

560 g des in Beispiel 8 hergestellten C₁₃-Oxoalkoholethoxilates werden in einem Rührgefäß unter Stickstoff auf 60°C erwärmt und langsam mit 92 g Polyphosphorsäure versetzt. Dabei sollte die Temperatur 65°C nicht übersteigen. Gegen Ende der Zugabe wird die Temperatur auf 70°C erhöht und eine Stunde bei dieser Temperatur gerührt.

Die Oberflächenspannung bei einer Konzentration von 1g/l beträgt 31,7 mN/m, gemessen nach DIN 53914.

### Beispiel 18, Herstellung eines Alkylsulfates

300 g C₁₃-Oxoalkohol (hergestellt durch Dimerisierung von 3-Hexen und anschließender Hydroformylierung) werden in einem Rührgefäß unter Stickstoff langsam mit 198 g Chlorschwefelsäure versetzt. Dabei sollte die Temperatur 30°C nicht übersteigen. Diese Mischung wird in eine Lösung von 68 g NaOH in 1060 ml Wasser gegeben

Die Oberflächenspannung bei einer Konzentration von 1g/l beträgt 36,1 mN/m, gemessen nach DIN 53914.

### Beispiel 19, Herstellung eines Alkylethersulfates

420 g des in Beispiel 8 hergestellten C₁₃-Oxoalkoholethoxilates werden in einem Rührgefäß unter Stickstoff auf 60°C erwärmt und langsam mit 198 g Chlorschwefelsäure versetzt. Dabei sollte die Temperatur 30°C nicht übersteigen. Diese Mischung wird in eine Lösung von 68 g NaOH in 1500 ml Wasser gegeben.

Die Oberflächenspannung bei einer Konzentration von 1g/l beträgt 33,1 mN/m, gemessen nach DIN 53914.

## Patentansprüche

1. Wasch- und Reinigungsmittel, umfassend einen wirksamen Anteil eines oder mehrerer Tenside der Formel I 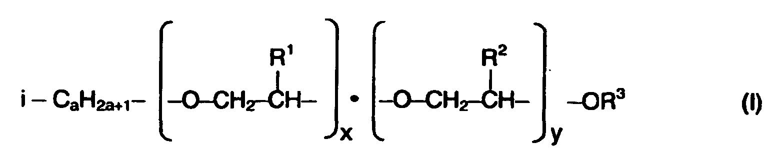 worin
a für eine der Zahlen 11, 12 und 13 steht, wobei der i - Cₐ H₂ₐ₊₁- Rest von einem Oxoalkohol abgeleitet ist, der durch Hydroformylierung eines Decens, **Undecens**, Dodecens oder eines Gemisches dieser Olefine erhalten wird, das seinerseits durch Dimerisierung von n-Penten-2, Hexen-3 oder von Gemischen dieser Verbindungen hergestellt worden ist,
R¹ und R² verschieden sind und jeweils Wasserstoff oder Alkylreste der Formel CₙH₂ₙ₊₁- bedeuten,
R³ Wasserstoff, oder einen Sulfato- oder Phosphatorest bedeutet,
n für eine Zahl von 1 bis 16, x für eine Zahl von 0 bis 200 und y für eine Zahl von 0 bis 200 steht,
sowie weitere in Wasch- und Reinigungsmitteln übliche bekannte Hilfs- und Zusatzstoffe und gegebenenfalls zusätzlich weitere Tenside.

2. Wasch- und Reinigungsmittel gemäß Anspruch 1, **dadurch gekennzeichnet, daß** der Mindestanteil der Tenside der Formel I am Gesamtgewicht der erfindungsgemäßen Wasch- und Reinigungsmittel so bemessen ist, daß sich eine signifikante Wirkung dieses Zusatzes zeigt.

3. Waschmittel gemäß Anspruch 2, **dadurch gekennzeichnet, daß** der Anteil der Tenside der Formel I in dem erfindungsgemäßen Waschmittel, bezogen auf das Gesamtgewicht des Mittels, 0,01 bis 50 Gew.-%, vorzugsweise 0,1 bis 40 Gew.-%, beträgt.

4. Reinigungsmittel gemäß Anspruch 2, **dadurch gekennzeichnet, daß** der Anteil der Tenside der Formel I in dem erfindungsgemäßen Reinigungsmittel, bezogen auf das Gesamtgewicht des Mittels, 0,01 bis 40 Gew.-%, vorzugsweise 0,1 bis 30 Gew.-%, beträgt.

5. Wasch- und Reinigungsmittel gemäß den Ansprüchen 1 bis 4 **dadurch gekennzeichnet, daß** in den Tensiden der Formel I a für den Wert 13 steht.

6. Wasch- und Reinigungsmittel gemäß den Ansprüchen 1 bis 5 **dadurch gekennzeichnet, daß** in den Tensiden der Formel I R¹ und R² verschieden sind und jeweils Wasserstoff, Methyl, Ethyl oder Propyl bedeuten.

7. Verfahren zur Herstellung von Wasch- und Reinigungsmitteln, **dadurch gekennzeichnet, daß** Tenside der Formel I eingesetzt werden.

8. Verwendung der Waschmittel des Anspruchs 1 in Waschverfahren.

9. Verwendung von Reinigungsmitteln des Anspruchs 1 zum Reinigen von geschlossenen, insbesondere von harten Oberflächen.

## Claims

1. A detergent or cleaner comprising an effective proportion of one or more surfactants of the formula I 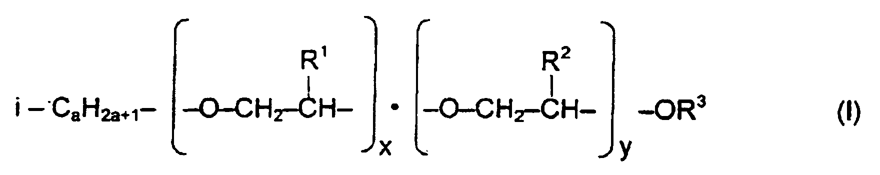 in which
a is one of the numbers 11, 12, or 13, where the i-CₐH₂ₐ₊₁ radical is derived from an oxo alcohol obtained by hydroformylation of a decene, **undecene** dodecene or a mixture of these olefins which, in turn, has been prepared by dimerizing n-pent-2-ene, hex-3-ene or mixtures of these compounds.
R¹ and R² are different and are each hydrogen or alkyl radicals of the formula CₙH₂ₙ₊₁-,
R³ is hydrogen, or a sulfato or phosphato radical,
n is a number from 1 to 16, x is a number from 0 to 200, and y is a number from 0 to 200,
and further known auxiliaries and additives customary in detergents and cleaners and
optionally in addition further surfactants.

2. A detergent or cleaner as claimed in claim 1, wherein the minimum proportion of the surfactants of the formula I based on the total weight of the detergent or cleaner according to the invention is such that the action of this additive is significant.

3. A detergent as claimed in claim 2, wherein the proportion of the surfactants of the formula I in the detergent according to the invention, based on the total weight of the composition, is 0.01 to 50% by weight, preferably 0.1 to 40% by weight.

4. A cleaner as claimed in claim 2, wherein the proportions of the surfactants of the formula I in the cleaner according to the invention, based on the total weight of the composition, is 0.01 to 40% by weight, preferably 0.1 to 30% by weight.

5. A detergent or cleaner as claimed in claims 1 to 4, wherein, in the surfactants of the formula I, a is 13.

6. A detergent or cleaner as claimed in claims 1 to 5, wherein, in the surfactants of the formula I, R¹ and R² are different and are each hydrogen, methyl, ethyl, or propyl.

7. A process for the preparation of detergents and cleaners, which comprises using surfactants of the formula I.

8. The use of the detergents of claim 1 in washing operations.

9. The use of cleaners of claim 1 for cleaning closed, in particular hard, surfaces.

## Revendications

1. Produit de lavage et de nettoyage, comprenant une fraction active d'un ou de plusieurs agents tensioactifs de la formule I : 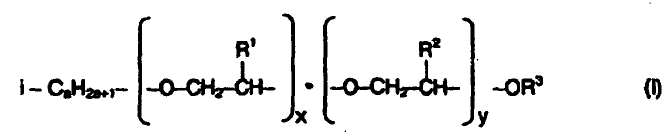 dans laquelle
a représente un des nombres 11, 12 et 13, le radical i - CₐH₂ₐ₊₁ étant dérivé d'un oxoalcool qui est obtenu par hydroformylation d'un décène, d'un undécène, d'un dodécène ou d'un mélange de ces oléfines, qui à son tour a été préparé par dimérisation de n-pentène-2, d'hexène-3 ou de mélanges de ces composés,
R¹ et R² sont différents et représentent chacun de l'hydrogène ou des radicaux alkyle de la formule CₙH₂ₙ₊₁-,
R³ représente de l'hydrogène ou un radical sulfato ou phosphato,
n représente un nombre de 1 à 16, x un nombre de 0 à 200 et y un nombre de 0 à 200,
ainsi que d'autres adjuvants et additifs connus, courants dans des produits de lavage et de nettoyage et éventuellement en supplément d'autres agents tensioactifs.

2. Produit de lavage et de nettoyage suivant la revendication 1, **caractérisé en ce que** la fraction minimale des agents tensioactifs de la formule I est mesurée sur le poids global du produit de lavage et de nettoyage suivant l'invention de façon qu'il apparaisse une action significative de cet additif.

3. Produit de lavage suivant la revendication 2, **caractérisé en ce que** la fraction des agents tensioactifs de la formule I dans le produit de lavage suivant l'invention est, par rapport au poids global du produit, de 0,01 à 50% en poids, de préférence de 0,1 à 40% en poids.

4. Produit de nettoyage suivant la revendication 2, **caractérisé en ce que** la fraction des agents tensioactifs de la formule I dans le produit de nettoyage suivant l'invention est, par rapport au poids global du produit, de 0,01 à 40% en poids, de préférence de 0,1 à 30% en poids.

5. Produit de lavage et de nettoyage suivant les revendications 1 à 4, **caractérisé en ce que**, dans les agents tensioactifs de la formule I, a représente la valeur 13.

6. Produit de lavage et de nettoyage suivant les revendications 1 à 5, **caractérisé en ce que**, dans les agents tensioactifs de la formule I, R¹ et R² sont différents et représentent respectivement de l'hydrogène, du méthyle, de l'éthyle ou du propyle.

7. Procédé de préparation de produits de lavage et de nettoyage, **caractérisé en ce qu'**on met en oeuvre des agents tensioactifs de la formule I.

8. Utilisation du produit de lavage de la revendication 1 dans un procédé de lavage.

9. Utilisation de produits de nettoyage de la revendication 1, pour nettoyer des surfaces fermées, en particulier des surfaces dures.
